# EUROPEAN PATENT APPLICATION

(11) **EP 3 246 028 A1**
(43) Date of publication of application: **22.11.2017**
(21) Application number: 16170166.9
(22) Date of filing: 18.05.2016
(51) Int. Cl.: A61K 31/47, C07D 215/56, C07D 401/04, A61P 3/10

(54) **QUINOLINE COMPOUNDS FOR INCREASING INSULINE RELEASE**

(71) Applicant: Max-Planck-Gesellschaft zur Förderung der Wissenschaften e.V., 80539 München (DE)
(72) Inventor: ÖRFI, Zoltan, 1161 Budapest (HU); ULLRICH, Axel, 80331 Munich (DE)
(74) Representative: Grünecker Patent- und Rechtsanwälte PartG mbB

(57) **Abstract**

The present invention relates to a class of quinoline compounds that act as insulin secretagogues, i.e., induce insulin secretion from beta cells. The present compounds may be used to induce divalent calcium cation influx in pancreatic beta-cells or to activate voltage-dependent calcium channels in pancreatic beta-cells. The present invention further relates to compounds for use in the treatment of diabetes.

## Description

### INTRODUCTION

Diabetes mellitus, commonly referred to as diabetes, is a highly prevalent and chronic metabolic disease that is characterized by high blood glucose levels. About 347 million people are diagnosed with diabetes worldwide and globally 6% of deaths are caused by diabetes. Untreated diabetes and therefore permanently increased levels of blood glucose, among other things increases the risk for coronary vascular disease, peripheral vascular disease, stroke, retinopathy, nephropathy and neuropathy. In general, diabetes is either due to insufficient amounts of the peptide hormone insulin, or due to a reduced sensitivity to insulin.

Insulin is a peptide hormone, which, in mammals including humans, is produced and secreted by beta cells in the pancreas. Beta cells are located in the so-called islets of Langerhans. Beta cells secrete insulin in response to elevated levels of blood glucose. Insulin in turn stimulates the glucose uptake of somatic cells, thereby reducing the blood glucose level.

Insulin consists of two polypeptide chains linked together by a disulfide bridge. Insulin is produced as a pre-pro peptide, which is then further matured inside the beta cells to the mature insulin peptide hormone. Insulin is secreted by the beta cells in response to elevated blood glucose levels. This release is regulated on multiple levels.

Glucose enters the beta cells through glucose transporters, such as GLUT2. Once inside the beta cells, glucose is metabolized and adenosine triphosphate (ATP) is produced. An increased intracellular ATP/ADP ratio leads to closure of ATP-sensitive Kir6.2/SUR1 potassium channels. Closure of the potassium channels leads to an increase of intracellular potassium, since passive diffusion of potassium ions into the extracellular milieu is blocked. Buildup of positively charged potassium ions inside the cell results in depolarization of the plasma membrane. Depolarization activates voltage-gated calcium ion channels, which allows calcium ions to diffuse into the beta cells. Finally, an increased intracellular concentration of calcium triggers the release of insulin-filled vesicles.

An alternative pathway for regulating insulin secretion is mediated by cyclic adenosine monophosphate (cAMP). cAMP is generated from ATP by the adenylyl cyclase (AC) enzyme. AC can be stimulated by the activation of various G-protein coupled receptors (GPCRs) that are found in beta cells (for example, GLP-1 receptor, beta-adrenergic receptor, GPR119). cAMP levels are negatively regulated by phosphodiesterase enzymes (PDE) which degrade cAMP. Increased intracellular cAMP levels potentiate exocytosis of insulin-filled vesicles, and thereby secretion of insulin, through different effector molecules (for example, PKA and Epac2). One of the GPCRs that stimulates AC activity, GLP-1 receptor, is bound and activated by the peptide hormone ligand glucagon like peptide-1 (GLP-1). GLP-1 this synthesized in the intestine in response to elevated levels of glucose and released into the bloodstream. By binding to GLP-1 receptor, GLP-1 promotes beta cell insulin secretion. In the bloodstream, GLP-1 is degraded by the enzyme dipeptidylpeptidase 4 (DPP 4). Thereby, DPP 4 indirectly negatively regulates beta cell insulin secretion.

Diabetes can be broadly categorized into two types, type I and type II diabetes. Type I diabetes (insulin-dependent diabetes or juvenile diabetes) is characterized by loss of insulin producing beta cells. Typically, loss of beta cells is due to an autoimmune reaction. Often, the disease manifests during childhood. Currently, type I diabetes treatment requires administration of insulin to the patient to accommodate for the loss of endogenous insulin (replacement therapy).

Type II diabetes is characterized by the body's reduced ability to respond to insulin, i.e., reduced insulin sensitivity. As a result, somatic cells have a reduced ability to take up glucose from the bloodstream and blood glucose levels are chronically elevated. The majority of diabetes patients suffer from type II diabetes. Diagnosis of type II diabetes is often more difficult, due to the slow progression of the disease. Due to the severe complications resulting from uncontrolled diabetes, early recognition of the disease is important. Oral glucose tolerance test (OGTT) is one possibility to diagnose the so called pre-diabetic state, i.e. an increased risk to develop type II diabetes. In some patients, reduced sensitivity to insulin can occur together with reduced insulin production and secretion by beta cells.

In contrast to type I diabetes, type II diabetes most often does not require replacement therapy with insulin. Instead, type II diabetes patients are often treated with a wide range of different, orally-available therapeutics. In general, anti-diabetic medication needs to be customized for every patient and combinations of drugs are sometimes necessary.

Currently, different medicaments are available for the treatment of type II diabetes, for example, insulin secretagogues, insulin sensitizers, DPP-4 inhibitors and SGLT 2 inhibitors.

Insulin secretagogues are a functionally defined class of therapeutics, whose members are characterized by their ability to stimulate insulin secretion in beta cells. One typical, representative compound family of the insulin secretagogue type is the sulfonylurea drug family. Sulfonylureas inhibit ATP-sensitive Kir6.2/SUR1 potassium channels by binding with high affinity to the SUR1 regulatory subunit. Blocking ATP-sensitive Kir6.2/SUR1 potassium channels induces membrane depolarization, which in turn promotes calcium influx into the beta cell, thereby essentially mimicking the effect of ATP. A major adverse reaction to sulfonylureas can be hypoglycemia, which in extreme cases can be life-threatening. Therefore, it is advised to take sulfonylureas during the meal. Weight gain is also associated with the long-term usage of sulfonylureas. Further, certain sulfonylureas might be potentially teratogenic. Permanent treatment with sulfonylureas can be associated with apoptosis and therefore loss of beta cell mass. First-generation sulfonylureas are chlorpropamide, tolbutamide and tolazamide. They are not recommended anymore and are rarely used due to severe adverse reactions. Second generation sulfonylureas, such as glipizide, glibenclamide/glyburide, gliquidone and glyclopyramide and third generation sulfonylureas, such as glimepiride, are still widely used for the treatment of type II diabetes.

Members of the meglitinide drug family promote insulin secretion by the same mechanism as sulfonylureas and therefore also belong to the insulin secretagogue type. Marketed members of this drug family are repaglinide, nateglinide and mitiglinide. They have a reduced tendency to cause hypoglycemia compared to sulfonylureas, however, hypoglycemia is still a potentially lethal adverse reaction associated with this class of compounds. Further, meglitinides are associated with weight gain.

Insulin sensitizers, on the other hand, act by reducing insulin resistance in somatic cells. Insulin sensitizers do not affect the amount of secreted insulin. Therefore, insulin sensitizers typically do not have the risk of inducing hypoglycemia. One representative class of insulin sensitizers is the biguanidine drug family, including, for example, metformin, buformin and phenformin. It is thought that they act by increasing glucose uptake in somatic cells, reducing glucose blood levels and reducing gluconeogenesis. Biguanidines have been associated with diarrhea, dyspepsia and lactic acidosis. Therefore, metformin, for example, is contraindicated in renal insufficiency. For these reasons, buformin and phenformin are practically not used anymore.

A second class of insulin sensitizers is the thiazolidinediones (TZD). Representatives of the TZD-family are rosiglitazone, pioglitazone, lobeglitazone and troglitazone. TZD-type insulin sensitizers act by activating peroxisome proliferator-activated receptors (PPAR), in particular PPAR-gamma. PPARs are nuclear receptors that are mainly expressed in brown adipose tissue and play a decisive role in the development of adipocytes. The endogenous ligands for these receptors are free fatty acids (FFAs) and eicosanoids. After activation of a PPAR, it forms a heterodimer with the retinoid receptor and acts as a transcription factor. TZDs have a complex effect that overall leads to improved insulin sensitivity of muscle and fat cells. Major adverse reactions associated with TZDs are coronary heart disease, edema, bladder cancer and hepatopathy.

As can be seen from the above, there is still an urgent need for new and improved small molecule drugs for the treatment of diabetes, due to the ever increasing incidence in the global population and the severe and sometimes even fatal adverse reactions associated with the presently available drugs.

It has been previously found that certain quinoline compounds that are typically used as antimalarial agents, for example, mefloquine and quinine, are able to induce insulin secretion in beta cells.

The present inventors have now surprisingly found that members of a structurally different class of quinoline compounds also act as insulin secretagogues, i.e., induce insulin secretion from beta cells. Further, the present inventors have surprisingly found that the quinoline compounds according to the present invention have improved properties compared to the previously reported quinoline compounds in the art with secretagogue activity.

### BRIEF SUMMARY OF THE INVENTION

In accordance with the above technical problem, the present invention relates to new insulin secretagogues in the form of small organic molecules having the activity of inducing insulin secretion in pancreatic beta cells, their use in medicine, their use in the treatment of diabetes, in particular type II diabetes, and their use in different in vitro applications.

In one aspect of the present invention an insulin secretagogue is provided having Formula I 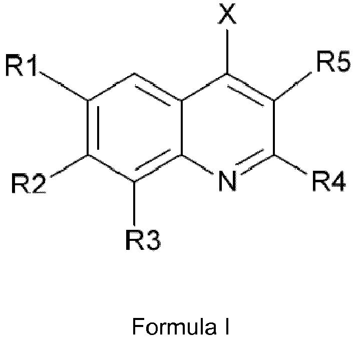 wherein X is selected from the group consisting of substituted or non-substituted phenyl, O-phenyl, N-phenyl, pyridyl, N-pyridyl, O-pyridyl, pyrimidyl, N-pyrimidyl, or O-pyrimidyl. R₁, R₂ and R₃ are independently selected from the group consisting of (a) hydrogen, (b) halogen, (c) substituted or non-substituted C1-C5 alkyl, (c) substituted or non-substituted, unsaturated C1-C5 alkyl, (d) hydroxyl, or (h) alkoxy (OY), wherein Y is selected from the group consisting of (a) a substituted or non-substituted C1-C5 alkyl, or (b) a substituted or non-substituted, unsaturated C1-C5 alkyl. R₄ and R₅ are independently selected from the group consisting of (a) hydrogen, (b) nitrile, (c) primary, secondary or tertiary amide, (c) carboxylic acid, (d) carboxylic acid ester, (e) methyl-N-methanesulfonamide or (f) alkylamine. The insulin secretagogue according to the present invention stimulates insulin secretion in pancreatic beta cells.

In another aspect the insulin secretagogue according to the present invention is provided for use in a therapeutic method.

In another aspect the insulin secretagogue according to the present invention is provided for use in the treatment of diabetes.

In yet another aspect, the invention relates to the use of the present insulin secretagogues for inducing insulin secretion in pancreatic beta cells in vitro, the use of the present insulin secretagogues for inducing divalent calcium cation influx in pancreatic beta cells in vitro, or the use of the present insulin secretagogues for activating voltage-dependent calcium channels in pancreatic beta cells in vitro.

### BRIEF DESCRIPTION OF THE FIGURES

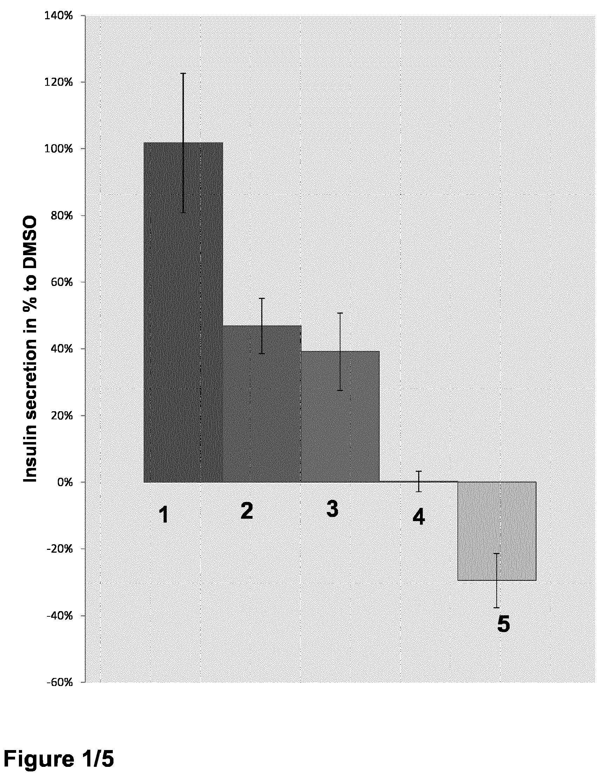

Figure 1: RIN5AH beta cells were treated with 5 µM compound 12 (1) (see Table 3) or 50 µM quinidine (2) or chloroquine (3), or 10 µM levofloxacin (4) or gatifloxacin (5) and insulin secretion was measured according to the procedure of Example 1 relative to a DMSO control. Compound 12 of the present invention is more effective in inducing insulin secretion in pancreatic beta cells compared to previously reported quinoline compounds quinidine, chloroquine, levofloxacin and gatifloxacin.

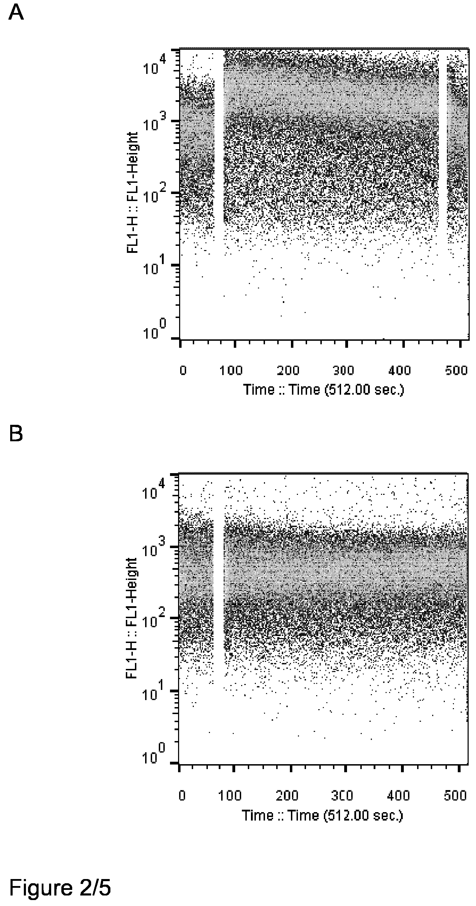

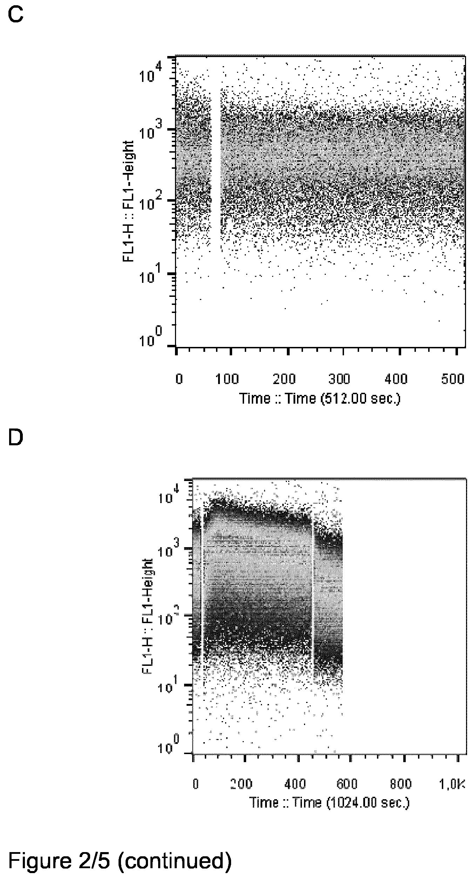

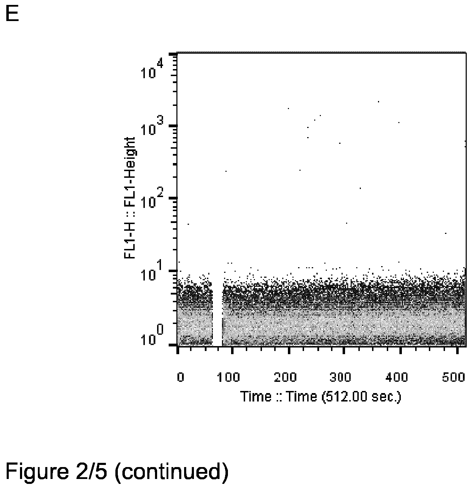

Figure 2: Calcium influx experiments by FACS. RIN5AH-cells were loaded with the calcium indicator Fluo-4 AM and incubated with different compounds according to the procedure described in Example 3. Calcium influx was analyzed by measuring Fluo-4 AM fluorescence by FACS analysis. The y-axis shows fluorescence intensity and the x-axis shows time in seconds. A, Addition of 5 µM compound 12 (see Table 3) (1st gap) induced Ca2+ influx in RIN-5AH cells when extracellular Ca2+ was present in the buffer. The long sustained signal could be quenched by EGTA (2nd gap). B-C, Two negative control quinoline compounds (compound 73, B, and compound 63, C) which didn't show any effect on insulin secretion and did not show any increase in calcium influx. D, Glibenclamide (GBA) effect. GBA, a sulfonylurea, also caused calcium influx, however, with a delayed response compared to compound 12. E, compound 12 did not change the fluorescence signal in Fluo-4 unstained cells. F-I, compounds 13 (F), 8 (G), 7 (H) and 18 (I) also induced Ca2+ influx.

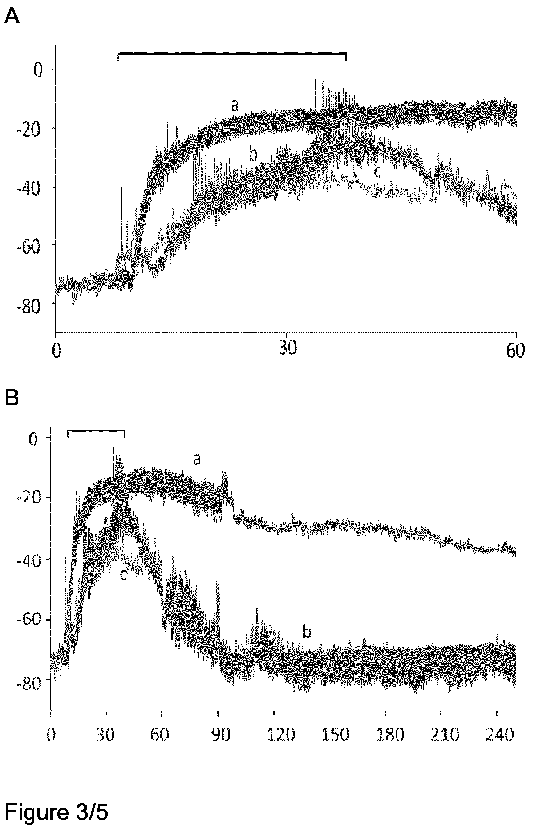

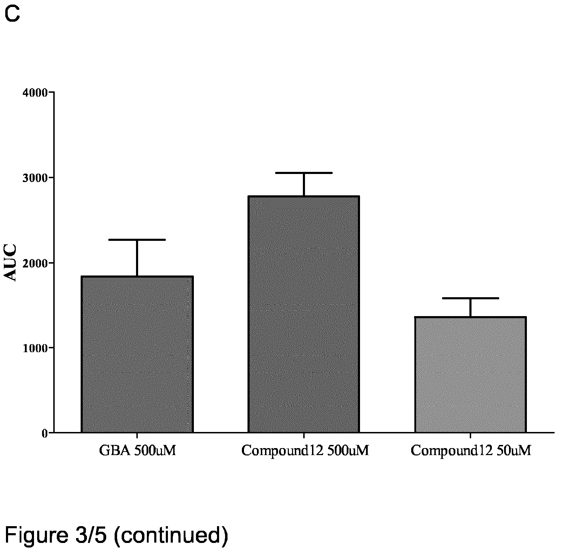

Figure 3: RIN-5AH cells were treated with compound 12 or GBA (glibenclamide) and membrane depolarization was assessed by current clamp measurements. Stimulations were averaged and compared to each other. A-B, x-axis shows time in seconds, y-axis shows membrane potential in mV. a: 500 µM compound 12, b: 500 µM GBA, c: 50 µM compound 12. Horizontal bar indicates a time window of 30 s during which compound 12 or GBA was applied. High concentration of compound 12 (500 µM) dramatically increased the depolarization rate in RIN-5AH cells and produced a sustained signal. C, Area under the curve (AUC; mV*s), calculated for 60 s period that correlates with the overall charge passing the membrane by stimulation (n=3-4).

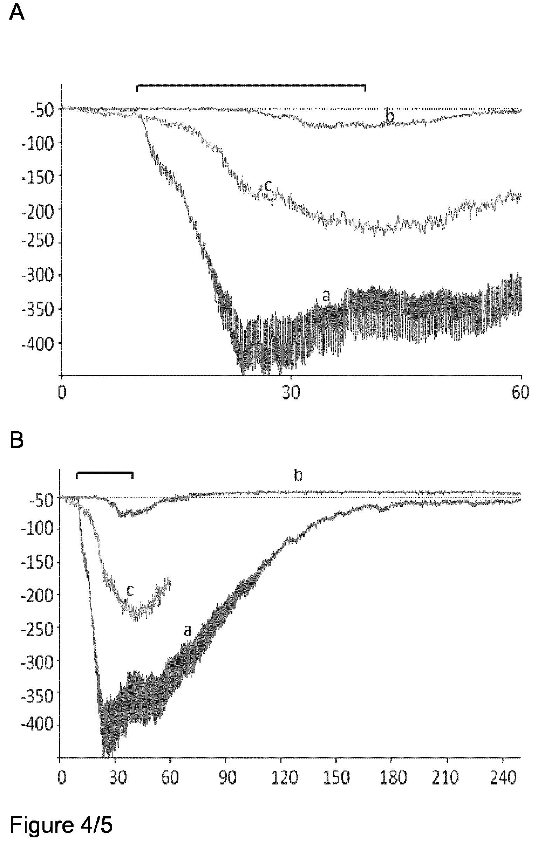

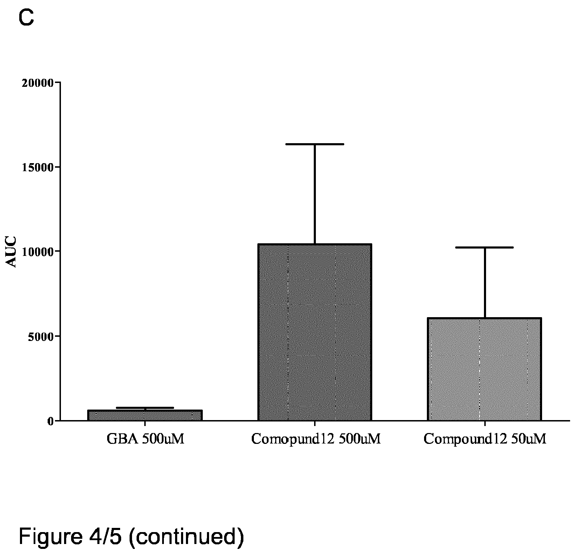

Figure 4: RIN-5AH cells were treated with compound 12 or GBA (glibenclamide) and ion currents were assessed by voltage clamp measurements. Stimulations were averaged and compared to each other. A-B, x-axis shows time in seconds, y-axis shows membrane currents in pA. a: 500 µM compound 12, b: 500 µM GBA, c: 50 µM compound 12. Horizontal bar indicates a time window of 30 s during which compound 12 or GBA was applied. Compound 12 has markedly increased the inward current in RIN-5AH cells and flow returned back to baseline after 3 minutes. Compound 12 had a longer lasting effect compared to GBA (glibenclamide). C, Area under the curve represents a quantitative comparison of total inward current calculated for 60 s period (AUC; pA*s) (n=3-4). The inward ion influx induced by compound treatments accounts for the increased intracellular calcium level.

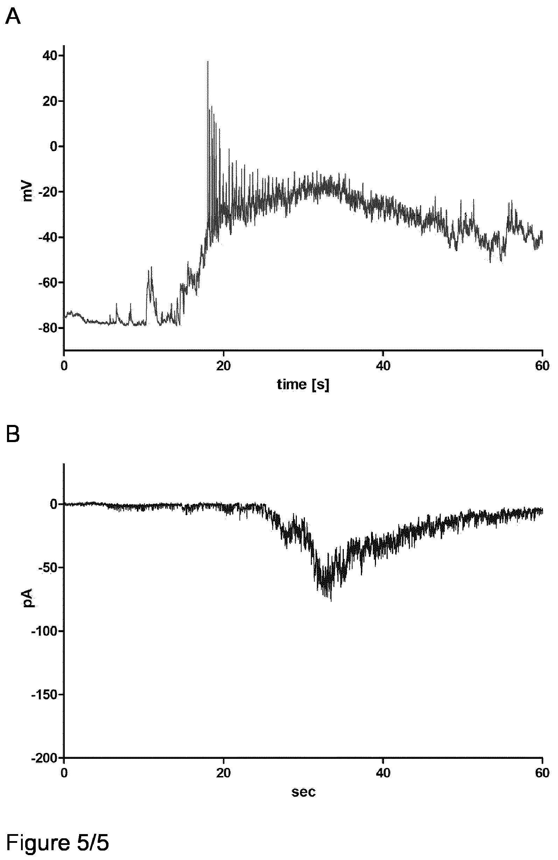

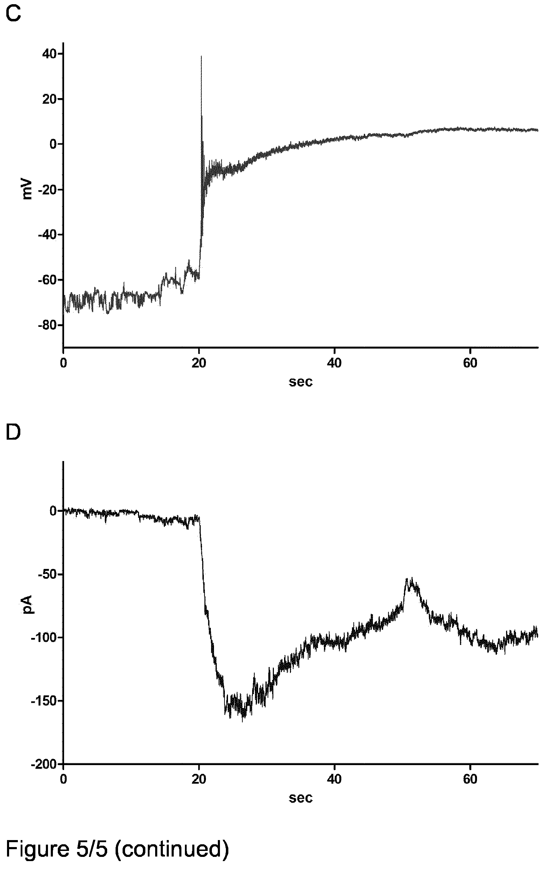

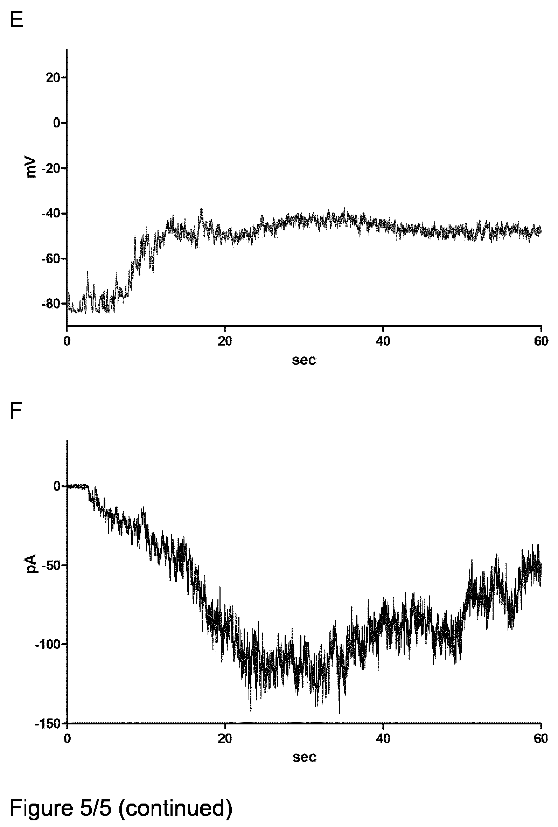

Figure 5: RIN-5AH cells were treated with compound 12 or GBA (glibenclamide) and voltage and current clamp analysis was conducted. Representative non-averaged whole cell patch clamp curves of RIN-5AH cells. The x-axis shows time in seconds, y-axis shows membrane potential in mV (current clamp) or ion currents in pA (voltage clamp). Horizontal bar indicates a time window of 30 s during which compound 12 or GBA was applied. A-B, Current clamp and voltage clamp measurements respectively for 500uM glibenclamide (GBA) was used as control in the experiments C-D, Current clamp and voltage clamp curves for 500uM compound 12. The effect in voltage clamp is immediate and not delayed like at glibenclamide treatment E-F, Effect of 50uM compound 12.

### GENERAL

The use of the term "comprising" as well as other grammatical forms such as "comprises" and "comprised" is not limiting. The terms "comprising", "comprises" and "comprised" should be understood as referring to an open-ended description of an embodiment of the present invention that may, but does not have to, include additional technical features in addition to the explicitly stated technical features. In the same sense the term "involving" as well as other respective grammatical forms such as "involves" and "involved" is not limiting. The same applies for the term "including" and other grammatical forms such as "includes" and "included". Section headings throughout the description are for organizational purposes only. In particular, they are not intended as limiting for various embodiments described therein, and it is to be understood that embodiments (and features therein) described under one subheading may be freely combined with embodiments (and features therein) described under another subheading.

In the forgoing and subsequent description of the present invention, any one embodiment is intended as being combinable with any other embodiments. Further, in the foregoing and subsequent description the features of any one embodiment are intended as being combinable with those in any other embodiment. Such combinations of one or more embodiments, and/or of one or more features in any one embodiment with one or more features in any other embodiment belong to the present invention and the present application is filed. The aforementioned is in particular true for the different substituents R₁, R₂, R₃, R₄, R₅ and X of Formula I listed above and below.

### DETAILED DESCRIPTION OF THE INVENTION

In one aspect of the present invention an insulin secretagogue is provided of Formula I 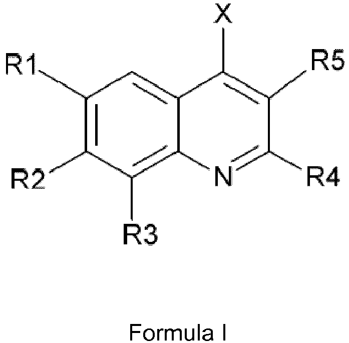 wherein X is selected from the group consisting of substituted or non-substituted phenyl, O-phenyl, N-phenyl, pyridyl, N-pyridyl, O-pyridyl, pyrimidyl, N-pyrimidyl, or O-pyrimidyl. R₁, R₂ and R₃ are independently selected from the group consisting of
(a) hydrogen,
(b) halogen,
(c) substituted or non-substituted C1-C5 alkyl,
(d) substituted or non-substituted, unsaturated C1-C5 alkyl,
(e) hydroxyl, or
(f) alkoxy (OY),
   wherein Y is selected from the group consisting of (i) a substituted or non-substituted C1-C5 alkyl, or ii) a substituted or non-substituted, unsaturated C1-C5 alkyl.
   R₄ and R₅ are independently selected from the group consisting of
   (a) hydrogen,
   (b) nitrile,
   (c) primary, secondary or tertiary amide,
   (d) carboxylic acid,
   (e) carboxylic acid ester,
   (f) methyl-N-methanesulfonamide or
   (g) alkylamine.

The insulin secretagogue according to the present invention directly stimulates insulin secretion in pancreatic beta cells.

In some embodiments of the present invention R₁ of Formula I is selected from the group consisting of (a) hydrogen, (b) fluorine, chlorine, or bromine, (c) methoxy, ethoxy, propoxy, butoxy, pentoxy, 3-aminopropoxy, N,N-dimethyl-3-aminopropoxy, 3-(morpholin-4-yl)propoxy, 3-hydroxypropoxy, 3-(pyrrolidin-1-yl)propoxy, 3-carbamidopropoxy, 3-acetamidopropoxy, 3-formamidopropoxy, N-3-phthalimidopropoxy, 4-aminobutoxy, N,N-dimethyl-4-aminobutoxy, 4-(morpholin-4-yl)butoxy, 4-hydroxybutoxy, 4-(pyrrolidin-1-yl)butoxy, 4-carbamidobutoxy, 4-acetamidobutoxy, 4-formamidobutoxy, N-4-phthalimidobutoxy or (d) trifluoromethyl or pentafluoroethyl.

Preferably, R₁ of Formula I is selected from the group consisting of hydrogen, fluorine, bromine, chlorine, methoxy, ethoxy, propoxy, 3-aminopropoxy, trifluoromethyl and 3-(morpholin-4-yl)propoxy.

More preferably, R₁ of Formula I is selected from the group consisting of hydrogen, fluorine, chlorine and methoxy. Most preferably, R₁ of Formula I is hydrogen or fluorine.

In some embodiments of the present invention R₂ of Formula I is selected from the group consisting of (a) hydrogen, (b) fluorine, chlorine, or bromine, (c) methoxy, ethoxy, propoxy, butoxy, pentoxy, 3-aminopropoxy, N,N-dimethyl-3-aminopropoxy, 3-(morpholin-4-yl)propoxy, 3-hydroxypropoxy, 3-(pyrrolidin-1-yl)propoxy, 3-carbamidopropoxy, 3-acetamidopropoxy, 3-formamidopropoxy, N-3-phthalimidopropoxy, 4-aminobutoxy, N,N-dimethyl-4-aminobutoxy, 4-(morpholin-4-yl)butoxy, 4-hydroxybutoxy, 4-(pyrrolidin-1-yl)butoxy, 4-carbamidobutoxy, 4-acetamidobutoxy, 4-formamidobutoxy, N-4-phthalimidobutoxy or (d) trifluoromethyl or pentafluoroethyl.

Preferably, R₂ of Formula I is selected from the group consisting of hydrogen, fluorine, bromine, chlorine, methoxy, ethoxy, propoxy, 3-aminopropoxy, trifluoromethyl, 3-(morpholin-4-yl)propoxy and N,N-dimethyl-3-aminopropoxy.

More preferably, R₂ of Formula I is selected from the group consisting of hydrogen, fluorine, methoxy, 3-aminopropoxy, 3-(morpholin-4-yl)propoxy and N,N-dimethyl-3-aminopropoxy.

Most preferably, R₂ of Formula I is hydrogen or fluorine.

In some embodiments of the present invention R₃ of Formula I is selected from the group consisting of (a) hydrogen, (b) fluorine, chlorine, or bromine, (c) methoxy, ethoxy, propoxy, butoxy, pentoxy, 3-aminopropoxy, N,N-dimethyl-3-aminopropoxy, 3-(morpholin-4-yl)propoxy, 3-hydroxypropoxy, 3-(pyrrolidin-1-yl)propoxy, 3-carbamidopropoxy, 3-acetamidopropoxy, 3-formamidopropoxy, N-3-phthalimidopropoxy, 4-aminobutoxy, N,N-dimethyl-4-aminobutoxy, 4-(morpholin-4-yl)butoxy, 4-hydroxybutoxy, 4-(pyrrolidin-1-yl)butoxy, 4-carbamidobutoxy, 4-acetamidobutoxy, 4-formamidobutoxy, N-4-phthalimidobutoxy or (d) trifluoromethyl or pentafluoroethyl.

Preferably, R₃ of Formula I is selected from the group consisting of hydrogen, fluorine, bromine, chlorine, methoxy and trifluoromethyl.

Most preferably, R₃ of Formula I is hydrogen, bromine, methoxy or fluorine.

In some embodiments of the present invention R₄ of Formula I is selected from the group consisting of (a) -CN, (b) -CONH₂, -CONHCH₃, or-CONH(CH₃)₂, (c) -COOH, -COOCH₃, or-COO(CH₃)₂, (d) -CH₂NH₂, -(CH₂)₂NH₂, or -(CH₂)₃NH₂, (e) -CH₂NSO₂CH₃ or (f) hydrogen.

Preferably, R₄ of Formula I is hydrogen, -CN or carbamido.

In some embodiments of the present invention R₅ of Formula I is selected from the group consisting of (a) -CN, (b) -CONH₂, -CONHCH₃, or -CONH(CH₃)₂, (c) -COOH, -COOCH₃, or-COO(CH₃)₂, (d) -CH₂NH₂, -(CH₂)₂NH₂, or -(CH₂)₃NH₂, (e) -CH₂NSO₂CH₃ or (f) hydrogen.

Preferably, R₅ of Formula I is hydrogen, or-CN.

In some embodiments of the present invention X of Formula I is selected from the group consisting of substituted or non-substituted phenyl, O-phenyl, N-phenyl, pyridyl, N-pyridyl, O-pyridyl, pyrimidyl, N-pyrimidyl, or O-pyrimidyl, wherein the phenyl, pyridyl or pyrimidyl is substituted with at least one substituent selected from the group consisting of
(a) halogen
(b) trifluoromethyl or pentafluoroethyl
(c) alkoxy
(e) primary, secondary or tertiary amide,
(f) primary, secondary, or tertiary alkylamine,
(g) substituted or non-substituted C1-C5 alkyl
(i) aryl (Ar), alkylaryl, heterocycle (Het), or alkylheterocycle,
(j) amine
(k) -NSO₂Ar, -NCOAr, -NCONAr, or -SO₂NAr.

In some embodiments of the present invention X of Formula I is selected from the group consisting of the variants listed in Table 1.

**Table 1 (the dot represents the quinoline ring of Formula I, to which substituent X is covalently bound):**

| **Number of Substituent X** | **Structure of Substituent X** |
|---|---|
| 1 | 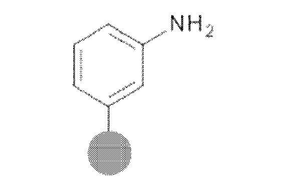 |
| 2 | 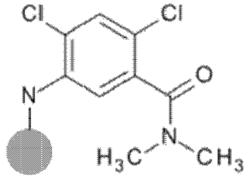 |
| 3 | 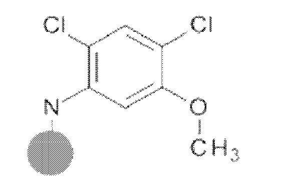 |
| 4 | 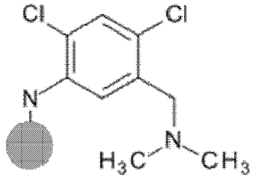 |
| 5 | 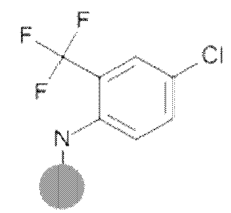 |
| 6 | 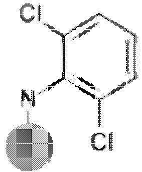 |
| 7 | 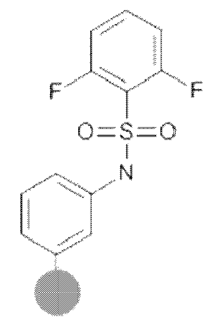 |
| 8 | 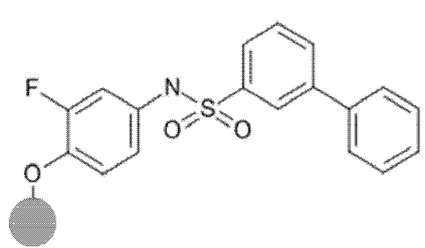 |
| 9 | 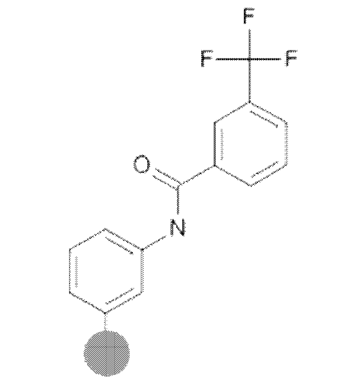 |
| 10 | 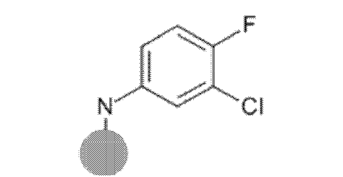 |
| 11 | 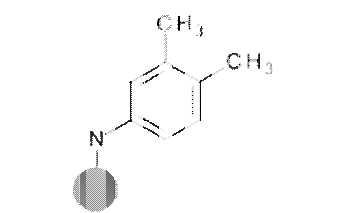 |
| 12 | 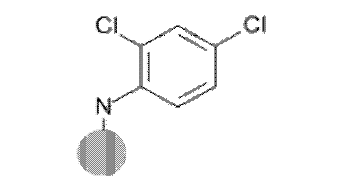 |
| 13 | 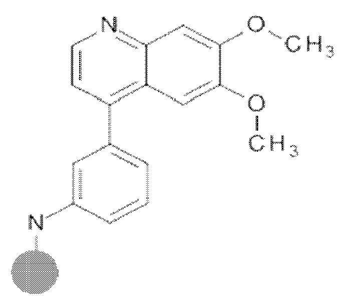 |
| 14 | 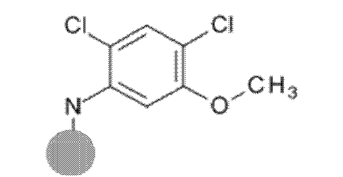 |
| 15 | 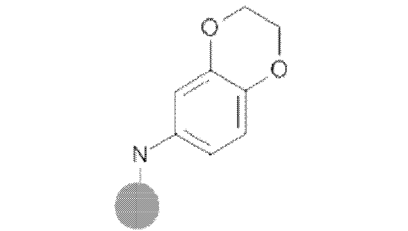 |
| 16 | 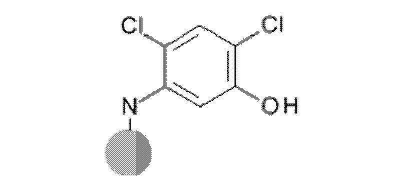 |
| 17 | 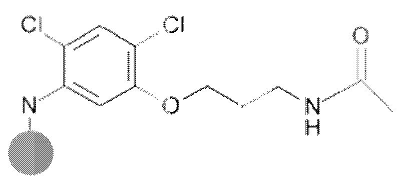 |
| 18 | 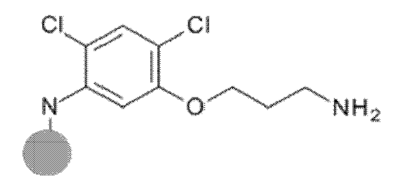 |
| 19 | 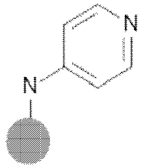 |
| 20 | 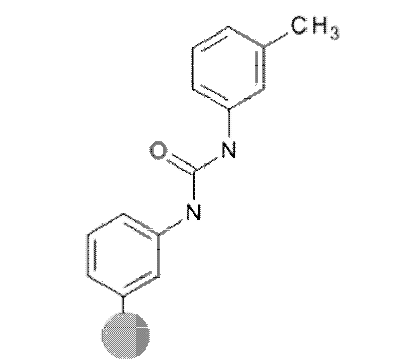 |
| 21 | 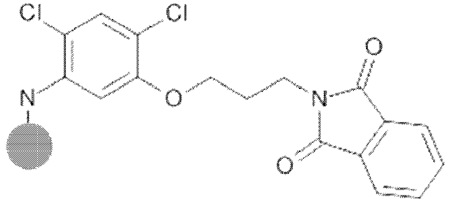 |
| 22 | 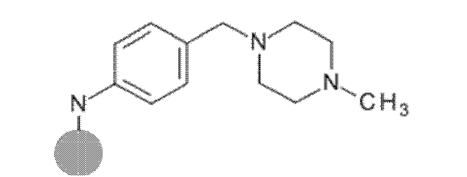 |
| 23 | 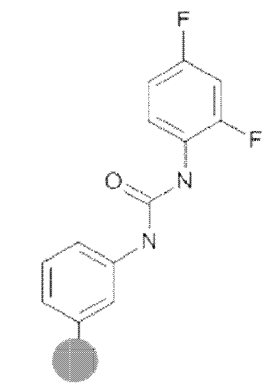 |
| 24 | 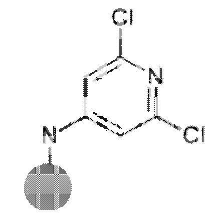 |
| 25 | 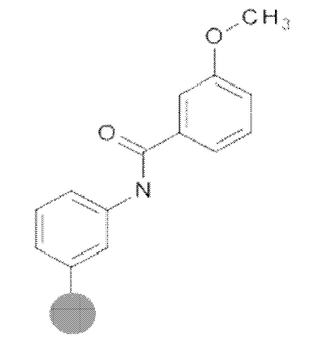 |
| 26 | 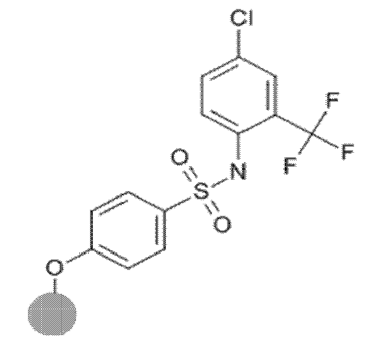 |
| 27 | 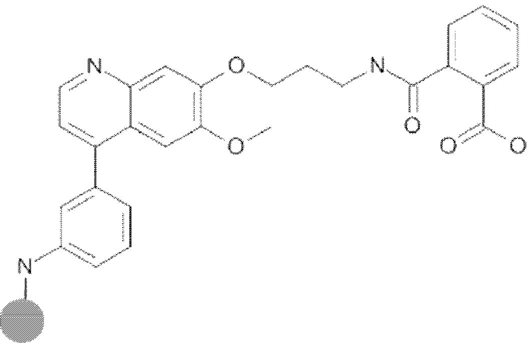 |
| 28 | 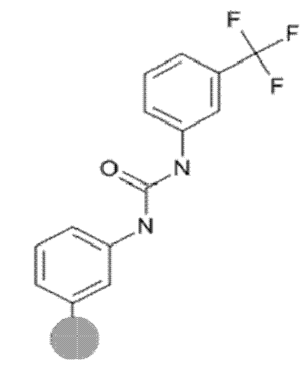 |

Preferably R₁, R₂ and R₃ of Formula I are independently selected from the group consisting of hydrogen, fluorine, bromine, methoxy, 3-aminopropoxy, chlorine, trifluoromethyl and 3-(morpholin-4-yl)propoxy.

In preferred embodiments of the present invention, R₁ is hydrogen and R₂ is fluorine, or R₁ and R₂ are both hydrogen, or R₁ is fluorine and R₂ is hydrogen, or R₁ is methoxy and R₂ is 3-aminopropoxy, or R₁ is methoxy and R₂ is fluorine, or R₁ is chlorine and R₂ is hydrogen, or R₁ is methoxy and R₂ is hydrogen. These combinations can be combined with any of the above or below disclosed substituents at the R3 position.

In preferred embodiments of the present invention, R₁ and R₂ are both hydrogen when R₃ is not hydrogen.

In further preferred embodiments of the present invention, R₁ is hydrogen and R₂ is fluorine when R₃ is not hydrogen.

In more preferred embodiments of the present invention, R₁ and R₂ are both hydrogen when R₃ is bromine, methoxy, fluorine or trifluoromethyl. In further preferred embodiments of the present invention, R₁ is hydrogen and R₂ is fluorine when R₃ is bromine, methoxy, fluorine or trifluoromethyl. Most preferably, R₁, R₂ and R₃ are respectively, hydrogen, fluorine and hydrogen, or hydrogen, hydrogen and bromine, or hydrogen, fluorine and methoxy, or fluorine, hydrogen and hydrogen, or hydrogen, hydrogen and fluorine, or methoxy, 3-aminopropoxy and hydrogen, or methoxy, fluorine and hydrogen, or chlorine, hydrogen and chorine, or methoxy, hydrogen and hydrogen.

This invention includes all possible combinations of the above listed substituents R₄ and R₅, which can be freely combined with the above listed embodiments concerning the substituents R₁, R₂ and R₃. Preferably, R₄ and R₅ are combined in that R₄ is nitrile and R₅ is hydrogen, or R₄ and R₅ are both hydrogen, or R₄ is hydrogen and R₅ is nitrile. Most preferably, R₄ is nitrile and R₅ is hydrogen. Even more preferred are embodiments wherein R₁, R₂, R₃, R₄ and R₅ are respectively, hydrogen, fluorine, hydrogen, nitrile and hydrogen, or hydrogen, hydrogen, bromine, nitrile and hydrogen, or hydrogen, fluorine, methoxy, nitrile and hydrogen, or fluorine, hydrogen, hydrogen, nitrile and hydrogen, or hydrogen, hydrogen, fluorine, nitrile and hydrogen, or methoxy, 3-aminopropoxy, hydrogen, nitrile and hydrogen, or methoxy, fluorine, hydrogen, nitrile and hydrogen, or chlorine, hydrogen, chorine, nitrile and hydrogen, or methoxy, hydrogen, hydrogen nitrile and hydrogen.

All of the above embodiments of substituent X can be combined with any of the embodiments of substituents R₁, R₂, R₃, R₄ and R₅ and in particular with any of the specifically disclosed combinations of R₁, R₂, R₃, R₄ and R₅, or any subgroups referred to in this application.

Preferred combinations of substituents R₁, R₂, R₃, R₄, R₅ and X of the insulin secretagogue of Formula I according to present invention are listed in Table 2.

**Table 2 ("halogen" of Table 2 can be fluorine, bromine, chlorine, or iodine; Y in "alkoxy (OY)" of Table 2 is selected from the group consisting of (a) a substituted or non-substituted C1-C5 alkyl, or (b) a substituted or non-substituted, unsaturated C1-C5 alkyl; preferably "alkoxy (OY)" of Table 2 can be methoxy or 3-aminopropoxy, most preferably it is methoxy; "X of Table 1" as listed in Table 2 means that substituent X can be selected from the group as listed in Table 1, "s/ns C1-C5 alkyl" means a substituted or non-substituted C1-C5 alkyl):**

| **R1** | **R2** | **R3** | **R4** | **R5** | **X** |
|---|---|---|---|---|---|
| H | H | H | H | H | X of Table 1 |
| H | H | H | H | -CN | X of Table 1 |
| H | H | H | H | -CONH2 | X of Table 1 |
| H | H | H | -CN | H | X of Table 1 |
| H | H | H | -CN | -CN | X of Table 1 |
| H | H | H | -CN | -CONH2 | X of Table 1 |
| H | H | H | -CONH2 | H | X of Table 1 |
| H | H | H | -CONH2 | -CN | X of Table 1 |
| H | H | H | -CONH2 | -CONH2 | X of Table 1 |
| H | H | halogen | H | H | X of Table 1 |
| H | H | halogen | H | -CN | X of Table 1 |
| H | H | halogen | H | -CONH2 | X of Table 1 |
| H | H | halogen | -CN | H | X of Table 1 |
| H | H | halogen | -CN | -CN | X of Table 1 |
| H | H | halogen | -CN | -CONH2 | X of Table 1 |
| H | H | halogen | -CONH2 | H | X of Table 1 |
| H | H | halogen | -CONH2 | -CN | X of Table 1 |
| H | H | halogen | -CONH2 | -CONH2 | X of Table 1 |
| H | H | alkoxy (OY) | H | H | X of Table 1 |
| H | H | alkoxy (OY) | H | -CN | X of Table 1 |
| H | H | alkoxy (OY) | H | -CONH2 | X of Table 1 |
| H | H | alkoxy (OY) | -CN | H | X of Table 1 |
| H | H | alkoxy (OY) | -CN | -CN | X of Table 1 |
| H | H | alkoxy (OY) | -CN | -CONH2 | X of Table 1 |
| H | H | alkoxy (OY) | -CONH2 | H | X of Table 1 |
| H | H | alkoxy (OY) | -CONH2 | -CN | X of Table 1 |
| H | H | alkoxy (OY) | -CONH2 | -CONH2 | X of Table 1 |
| H | H | s/ns C1-C5 alkyl | H | H | X of Table 1 |
| H | H | s/ns C1-C5 alkyl | H | -CN | X of Table 1 |
| H | H | s/ns C1-C5 alkyl | H | -CONH2 | X of Table 1 |
| H | H | s/ns C1-C5 alkyl | -CN | H | X of Table 1 |
| H | H | s/ns C1-C5 alkyl | -CN | -CN | X of Table 1 |
| H | H | s/ns C1-C5 alkyl | -CN | -CONH2 | X of Table 1 |
| H | H | s/ns C1-C5 alkyl | -CONH2 | H | X of Table 1 |
| H | H | s/ns C1-C5 alkyl | -CONH2 | -CN | X of Table 1 |
| H | H | s/ns C1-C5 alkyl | -CONH2 | -CONH2 | X of Table 1 |
| H | halogen | H | H | H | X of Table 1 |
| H | halogen | H | H | -CN | X of Table 1 |
| H | halogen | H | H | -CONH2 | X of Table 1 |
| H | halogen | H | -CN | H | X of Table 1 |
| H | halogen | H | -CN | -CN | X of Table 1 |
| H | halogen | H | -CN | -CONH2 | X of Table 1 |
| H | halogen | H | -CONH2 | H | X of Table 1 |
| H | halogen | H | -CONH2 | -CN | X of Table 1 |
| H | halogen | H | -CONH2 | -CONH2 | X of Table 1 |
| H | halogen | halogen | H | H | X of Table 1 |
| H | halogen | halogen | H | -CN | X of Table 1 |
| H | halogen | halogen | H | -CONH2 | X of Table 1 |
| H | halogen | halogen | -CN | H | X of Table 1 |
| H | halogen | halogen | -CN | -CN | X of Table 1 |
| H | halogen | halogen | -CN | -CONH2 | X of Table 1 |
| H | halogen | halogen | -CONH2 | H | X of Table 1 |
| H | halogen | halogen | -CONH2 | -CN | X of Table 1 |
| H | halogen | halogen | -CONH2 | -CONH2 | X of Table 1 |
| H | halogen | alkoxy (OY) | H | H | X of Table 1 |
| H | halogen | alkoxy (OY) | H | -CN | X of Table 1 |
| H | halogen | alkoxy (OY) | H | -CONH2 | X of Table 1 |
| H | halogen | alkoxy (OY) | -CN | H | X of Table 1 |
| H | halogen | alkoxy (OY) | -CN | -CN | X of Table 1 |
| H | halogen | alkoxy (OY) | -CN | -CONH2 | X of Table 1 |
| H | halogen | alkoxy (OY) | -CONH2 | H | X of Table 1 |
| H | halogen | alkoxy (OY) | -CONH2 | -CN | X of Table 1 |
| H | halogen | alkoxy (OY) | -CONH2 | -CONH2 | X of Table 1 |
| H | halogen | s/ns C1-C5 alkyl | H | H | X of Table 1 |
| H | halogen | s/ns C1-C5 alkyl | H | -CN | X of Table 1 |
| H | halogen | s/ns C1-C5 alkyl | H | -CONH2 | X of Table 1 |
| H | halogen | s/ns C1-C5 alkyl | -CN | H | X of Table 1 |
| H | halogen | s/ns C1-C5 alkyl | -CN | -CN | X of Table 1 |
| H | halogen | s/ns C1-C5 alkyl | -CN | -CONH2 | X of Table 1 |
| H | halogen | s/ns C1-C5 alkyl | -CONH2 | H | X of Table 1 |
| H | halogen | s/ns C1-C5 alkyl | -CONH2 | -CN | X of Table 1 |
| H | halogen | s/ns C1-C5 alkyl | -CONH2 | -CONH2 | X of Table 1 |
| H | alkoxy (OY) | H | H | H | X of Table 1 |
| H | alkoxy (OY) | H | H | -CN | X of Table 1 |
| H | alkoxy (OY) | H | H | -CONH2 | X of Table 1 |
| H | alkoxy (OY) | H | -CN | H | X of Table 1 |
| H | alkoxy (OY) | H | -CN | -CN | X of Table 1 |
| H | alkoxy (OY) | H | -CN | -CONH2 | X of Table 1 |
| H | alkoxy (OY) | H | -CONH2 | H | X of Table 1 |
| H | alkoxy (OY) | H | -CONH2 | -CN | X of Table 1 |
| H | alkoxy (OY) | H | -CONH2 | -CONH2 | X of Table 1 |
| H | alkoxy (OY) | halogen | H | H | X of Table 1 |
| H | alkoxy (OY) | halogen | H | -CN | X of Table 1 |
| H | alkoxy (OY) | halogen | H | -CONH2 | X of Table 1 |
| H | alkoxy (OY) | halogen | -CN | H | X of Table 1 |
| H | alkoxy (OY) | halogen | -CN | -CN | X of Table 1 |
| H | alkoxy (OY) | halogen | -CN | -CONH2 | X of Table 1 |
| H | alkoxy (OY) | halogen | -CONH2 | H | X of Table 1 |
| H | alkoxy (OY) | halogen | -CONH2 | -CN | X of Table 1 |
| H | alkoxy (OY) | halogen | -CONH2 | -CONH2 | X of Table 1 |
| H | alkoxy (OY) | alkoxy (OY) | H | H | X of Table 1 |
| H | alkoxy (OY) | alkoxy (OY) | H | -CN | X of Table 1 |
| H | alkoxy (OY) | alkoxy (OY) | H | -CONH2 | X of Table 1 |
| H | alkoxy (OY) | alkoxy (OY) | -CN | H | X of Table 1 |
| H | alkoxy (OY) | alkoxy (OY) | -CN | -CN | X of Table 1 |
| H | alkoxy (OY) | alkoxy (OY) | -CN | -CONH2 | X of Table 1 |
| H | alkoxy (OY) | alkoxy (OY) | -CONH2 | H | X of Table 1 |
| H | alkoxy (OY) | alkoxy (OY) | -CONH2 | -CN | X of Table 1 |
| H | alkoxy (OY) | alkoxy (OY) | -CONH2 | -CONH2 | X of Table 1 |
| H | alkoxy (OY) | s/ns C1-C5 alkyl | H | H | X of Table 1 |
| H | alkoxy (OY) | s/ns C1-C5 alkyl | H | -CN | X of Table 1 |
| H | alkoxy (OY) | s/ns C1-C5 alkyl | H | -CONH2 | X of Table 1 |
| H | alkoxy (OY) | s/ns C1-C5 alkyl | -CN | H | X of Table 1 |
| H | alkoxy (OY) | s/ns C1-C5 alkyl | -CN | -CN | X of Table 1 |
| H | alkoxy (OY) | s/ns C1-C5 alkyl | -CN | -CONH2 | X of Table 1 |
| H | alkoxy (OY) | s/ns C1-C5 alkyl | -CONH2 | H | X of Table 1 |
| H | alkoxy (OY) | s/ns C1-C5 alkyl | -CONH2 | -CN | X of Table 1 |
| H | alkoxy (OY) | s/ns C1-C5 alkyl | -CONH2 | -CONH2 | X of Table 1 |
| halogen | H | H | H | H | X of Table 1 |
| halogen | H | H | H | -CN | X of Table 1 |
| halogen | H | H | H | -CONH2 | X of Table 1 |
| halogen | H | H | -CN | H | X of Table 1 |
| halogen | H | H | -CN | -CN | X of Table 1 |
| halogen | H | H | -CN | -CONH2 | X of Table 1 |
| halogen | H | H | -CONH2 | H | X of Table 1 |
| halogen | H | H | -CONH2 | -CN | X of Table 1 |
| halogen | H | H | -CONH2 | -CONH2 | X of Table 1 |
| halogen | H | halogen | H | H | X of Table 1 |
| halogen | H | halogen | H | -CN | X of Table 1 |
| halogen | H | halogen | H | -CONH2 | X of Table 1 |
| halogen | H | halogen | -CN | H | X of Table 1 |
| halogen | H | halogen | -CN | -CN | X of Table 1 |
| halogen | H | halogen | -CN | -CONH2 | X of Table 1 |
| halogen | H | halogen | -CONH2 | H | X of Table 1 |
| halogen | H | halogen | -CONH2 | -CN | X of Table 1 |
| halogen | H | halogen | -CONH2 | -CONH2 | X of Table 1 |
| halogen | H | alkoxy (OY) | H | H | X of Table 1 |
| halogen | H | alkoxy (OY) | H | -CN | X of Table 1 |
| halogen | H | alkoxy (OY) | H | -CONH2 | X of Table 1 |
| halogen | H | alkoxy (OY) | -CN | H | X of Table 1 |
| halogen | H | alkoxy (OY) | -CN | -CN | X of Table 1 |
| halogen | H | alkoxy (OY) | -CN | -CONH2 | X of Table 1 |
| halogen | H | alkoxy (OY) | -CONH2 | H | X of Table 1 |
| halogen | H | alkoxy (OY) | -CONH2 | -CN | X of Table 1 |
| halogen | H | alkoxy (OY) | -CONH2 | -CONH2 | X of Table 1 |
| halogen | H | s/ns C1-C5 alkyl | H | H | X of Table 1 |
| halogen | H | s/ns C1-C5 alkyl | H | -CN | X of Table 1 |
| halogen | H | s/ns C1-C5 alkyl | H | -CONH2 | X of Table 1 |
| halogen | H | s/ns C1-C5 alkyl | -CN | H | X of Table 1 |
| halogen | H | s/ns C1-C5 alkyl | -CN | -CN | X of Table 1 |
| halogen | H | s/ns C1-C5 alkyl | -CN | -CONH2 | X of Table 1 |
| halogen | H | s/ns C1-C5 alkyl | -CONH2 | H | X of Table 1 |
| halogen | H | s/ns C1-C5 alkyl | -CONH2 | -CN | X of Table 1 |
| halogen | H | s/ns C1-C5 alkyl | -CONH2 | -CONH2 | X of Table 1 |
| halogen | halogen | H | H | H | X of Table 1 |
| halogen | halogen | H | H | -CN | X of Table 1 |
| halogen | halogen | H | H | -CONH2 | X of Table 1 |
| halogen | halogen | H | -CN | H | X of Table 1 |
| halogen | halogen | H | -CN | -CN | X of Table 1 |
| halogen | halogen | H | -CN | -CONH2 | X of Table 1 |
| halogen | halogen | H | -CONH2 | H | X of Table 1 |
| halogen | halogen | H | -CONH2 | -CN | X of Table 1 |
| halogen | halogen | H | -CONH2 | -CONH2 | X of Table 1 |
| halogen | halogen | halogen | H | H | X of Table 1 |
| halogen | halogen | halogen | H | -CN | X of Table 1 |
| halogen | halogen | halogen | H | -CONH2 | X of Table 1 |
| halogen | halogen | halogen | -CN | H | X of Table 1 |
| halogen | halogen | halogen | -CN | -CN | X of Table 1 |
| halogen | halogen | halogen | -CN | -CONH2 | X of Table 1 |
| halogen | halogen | halogen | -CONH2 | H | X of Table 1 |
| halogen | halogen | halogen | -CONH2 | -CN | X of Table 1 |
| halogen | halogen | halogen | -CONH2 | -CONH2 | X of Table 1 |
| halogen | halogen | alkoxy (OY) | H | H | X of Table 1 |
| halogen | halogen | alkoxy (OY) | H | -CN | X of Table 1 |
| halogen | halogen | alkoxy (OY) | H | -CONH2 | X of Table 1 |
| halogen | halogen | alkoxy (OY) | -CN | H | X of Table 1 |
| halogen | halogen | alkoxy (OY) | -CN | -CN | X of Table 1 |
| halogen | halogen | alkoxy (OY) | -CN | -CONH2 | X of Table 1 |
| halogen | halogen | alkoxy (OY) | -CONH2 | H | X of Table 1 |
| halogen | halogen | alkoxy (OY) | -CONH2 | -CN | X of Table 1 |
| halogen | halogen | alkoxy (OY) | -CONH2 | -CONH2 | X of Table 1 |
| halogen | halogen | s/ns C1-C5 alkyl | H | H | X of Table 1 |
| halogen | halogen | s/ns C1-C5 alkyl | H | -CN | X of Table 1 |
| halogen | halogen | s/ns C1-C5 alkyl | H | -CONH2 | X of Table 1 |
| halogen | halogen | s/ns C1-C5 alkyl | -CN | H | X of Table 1 |
| halogen | halogen | s/ns C1-C5 alkyl | -CN | -CN | X of Table 1 |
| halogen | halogen | s/ns C1-C5 alkyl | -CN | -CONH2 | X of Table 1 |
| halogen | halogen | s/ns C1-C5 alkyl | -CONH2 | H | X of Table 1 |
| halogen | halogen | s/ns C1-C5 alkyl | -CONH2 | -CN | X of Table 1 |
| halogen | halogen | s/ns C1-C5 alkyl | -CONH2 | -CONH2 | X of Table 1 |
| halogen | alkoxy (OY) | H | H | H | X of Table 1 |
| halogen | alkoxy (OY) | H | H | -CN | X of Table 1 |
| halogen | alkoxy (OY) | H | H | -CONH2 | X of Table 1 |
| halogen | alkoxy (OY) | H | -CN | H | X of Table 1 |
| halogen | alkoxy (OY) | H | -CN | -CN | X of Table 1 |
| halogen | alkoxy (OY) | H | -CN | -CONH2 | X of Table 1 |
| halogen | alkoxy (OY) | H | -CONH2 | H | X of Table 1 |
| halogen | alkoxy (OY) | H | -CONH2 | -CN | X of Table 1 |
| halogen | alkoxy (OY) | H | -CONH2 | -CONH2 | X of Table 1 |
| halogen | alkoxy (OY) | halogen | H | H | X of Table 1 |
| halogen | alkoxy (OY) | halogen | H | -CN | X of Table 1 |
| halogen | alkoxy (OY) | halogen | H | -CONH2 | X of Table 1 |
| halogen | alkoxy (OY) | halogen | -CN | H | X of Table 1 |
| halogen | alkoxy (OY) | halogen | -CN | -CN | X of Table 1 |
| halogen | alkoxy (OY) | halogen | -CN | -CONH2 | X of Table 1 |
| halogen | alkoxy (OY) | halogen | -CONH2 | H | X of Table 1 |
| halogen | alkoxy (OY) | halogen | -CONH2 | -CN | X of Table 1 |
| halogen | alkoxy (OY) | halogen | -CONH2 | -CONH2 | X of Table 1 |
| halogen | alkoxy (OY) | alkoxy (OY) | H | H | X of Table 1 |
| halogen | alkoxy (OY) | alkoxy (OY) | H | -CN | X of Table 1 |
| halogen | alkoxy (OY) | alkoxy (OY) | H | -CONH2 | X of Table 1 |
| halogen | alkoxy (OY) | alkoxy (OY) | -CN | H | X of Table 1 |
| halogen | alkoxy (OY) | alkoxy (OY) | -CN | -CN | X of Table 1 |
| halogen | alkoxy (OY) | alkoxy (OY) | -CN | -CONH2 | X of Table 1 |
| halogen | alkoxy (OY) | alkoxy (OY) | -CONH2 | H | X of Table 1 |
| halogen | alkoxy (OY) | alkoxy (OY) | -CONH2 | -CN | X of Table 1 |
| halogen | alkoxy (OY) | alkoxy (OY) | -CONH2 | -CONH2 | X of Table 1 |
| halogen | alkoxy (OY) | s/ns C1-C5 alkyl | H | H | X of Table 1 |
| halogen | alkoxy (OY) | s/ns C1-C5 alkyl | H | -CN | X of Table 1 |
| halogen | alkoxy (OY) | s/ns C1-C5 alkyl | H | -CONH2 | X of Table 1 |
| halogen | alkoxy (OY) | s/ns C1-C5 alkyl | -CN | H | X of Table 1 |
| halogen | alkoxy (OY) | s/ns C1-C5 alkyl | -CN | -CN | X of Table 1 |
| halogen | alkoxy (OY) | s/ns C1-C5 alkyl | -CN | -CONH2 | X of Table 1 |
| halogen | alkoxy (OY) | s/ns C1-C5 alkyl | -CONH2 | H | X of Table 1 |
| halogen | alkoxy (OY) | s/ns C1-C5 alkyl | -CONH2 | -CN | X of Table 1 |
| halogen | alkoxy (OY) | s/ns C1-C5 alkyl | -CONH2 | -CONH2 | X of Table 1 |
| alkoxy (OY) | H | H | H | H | X of Table 1 |
| alkoxy (OY) | H | H | H | -CN | X of Table 1 |
| alkoxy (OY) | H | H | H | -CONH2 | X of Table 1 |
| alkoxy (OY) | H | H | -CN | H | X of Table 1 |
| alkoxy (OY) | H | H | -CN | -CN | X of Table 1 |
| alkoxy (OY) | H | H | -CN | -CONH2 | X of Table 1 |
| alkoxy (OY) | H | H | -CONH2 | H | X of Table 1 |
| alkoxy (OY) | H | H | -CONH2 | -CN | X of Table 1 |
| alkoxy (OY) | H | H | -CONH2 | -CONH2 | X of Table 1 |
| alkoxy (OY) | H | halogen | H | H | X of Table 1 |
| alkoxy (OY) | H | halogen | H | -CN | X of Table 1 |
| alkoxy (OY) | H | halogen | H | -CONH2 | X of Table 1 |
| alkoxy (OY) | H | halogen | -CN | H | X of Table 1 |
| alkoxy (OY) | H | halogen | -CN | -CN | X of Table 1 |
| alkoxy (OY) | H | halogen | -CN | -CONH2 | X of Table 1 |
| alkoxy (OY) | H | halogen | -CONH2 | H | X of Table 1 |
| alkoxy (OY) | H | halogen | -CONH2 | -CN | X of Table 1 |
| alkoxy (OY) | H | halogen | -CONH2 | -CONH2 | X of Table 1 |
| alkoxy (OY) | H | alkoxy (OY) | H | H | X of Table 1 |
| alkoxy (OY) | H | alkoxy (OY) | H | -CN | X of Table 1 |
| alkoxy (OY) | H | alkoxy (OY) | H | -CONH2 | X of Table 1 |
| alkoxy (OY) | H | alkoxy (OY) | -CN | H | X of Table 1 |
| alkoxy (OY) | H | alkoxy (OY) | -CN | -CN | X of Table 1 |
| alkoxy (OY) | H | alkoxy (OY) | -CN | -CONH2 | X of Table 1 |
| alkoxy (OY) | H | alkoxy (OY) | -CONH2 | H | X of Table 1 |
| alkoxy (OY) | H | alkoxy (OY) | -CONH2 | -CN | X of Table 1 |
| alkoxy (OY) | H | alkoxy (OY) | -CONH2 | -CONH2 | X of Table 1 |
| alkoxy (OY) | H | s/ns C1-C5 alkyl | H | H | X of Table 1 |
| alkoxy (OY) | H | s/ns C1-C5 alkyl | H | -CN | X of Table 1 |
| alkoxy (OY) | H | s/ns C1-C5 alkyl | H | -CONH2 | X of Table 1 |
| alkoxy (OY) | H | s/ns C1-C5 alkyl | -CN | H | X of Table 1 |
| alkoxy (OY) | H | s/ns C1-C5 alkyl | -CN | -CN | X of Table 1 |
| alkoxy (OY) | H | s/ns C1-C5 alkyl | -CN | -CONH2 | X of Table 1 |
| alkoxy (OY) | H | s/ns C1-C5 alkyl | -CONH2 | H | X of Table 1 |
| alkoxy (OY) | H | s/ns C1-C5 alkyl | -CONH2 | -CN | X of Table 1 |
| alkoxy (OY) | H | s/ns C1-C5 alkyl | -CONH2 | -CONH2 | X of Table 1 |
| alkoxy (OY) | halogen | H | H | H | X of Table 1 |
| alkoxy (OY) | halogen | H | H | -CN | X of Table 1 |
| alkoxy (OY) | halogen | H | H | -CONH2 | X of Table 1 |
| alkoxy (OY) | halogen | H | -CN | H | X of Table 1 |
| alkoxy (OY) | halogen | H | -CN | -CN | X of Table 1 |
| alkoxy (OY) | halogen | H | -CN | -CONH2 | X of Table 1 |
| alkoxy (OY) | halogen | H | -CONH2 | H | X of Table 1 |
| alkoxy (OY) | halogen | H | -CONH2 | -CN | X of Table 1 |
| alkoxy (OY) | halogen | H | -CONH2 | -CONH2 | X of Table 1 |
| alkoxy (OY) | halogen | halogen | H | H | X of Table 1 |
| alkoxy (OY) | halogen | halogen | H | -CN | X of Table 1 |
| alkoxy (OY) | halogen | halogen | H | -CONH2 | X of Table 1 |
| alkoxy (OY) | halogen | halogen | -CN | H | X of Table 1 |
| alkoxy (OY) | halogen | halogen | -CN | -CN | X of Table 1 |
| alkoxy (OY) | halogen | halogen | -CN | -CONH2 | X of Table 1 |
| alkoxy (OY) | halogen | halogen | -CONH2 | H | X of Table 1 |
| alkoxy (OY) | halogen | halogen | -CONH2 | -CN | X of Table 1 |
| alkoxy (OY) | halogen | halogen | -CONH2 | -CONH2 | X of Table 1 |
| alkoxy (OY) | halogen | alkoxy (OY) | H | H | X of Table 1 |
| alkoxy (OY) | halogen | alkoxy (OY) | H | -CN | X of Table 1 |
| alkoxy (OY) | halogen | alkoxy (OY) | H | -CONH2 | X of Table 1 |
| alkoxy (OY) | halogen | alkoxy (OY) | -CN | H | X of Table 1 |
| alkoxy (OY) | halogen | alkoxy (OY) | -CN | -CN | X of Table 1 |
| alkoxy (OY) | halogen | alkoxy (OY) | -CN | -CONH2 | X of Table 1 |
| alkoxy (OY) | halogen | alkoxy (OY) | -CONH2 | H | X of Table 1 |
| alkoxy (OY) | halogen | alkoxy (OY) | -CONH2 | -CN | X of Table 1 |
| alkoxy (OY) | halogen | alkoxy (OY) | -CONH2 | -CONH2 | X of Table 1 |
| alkoxy (OY) | halogen | s/ns C1-C5 alkyl | H | H | X of Table 1 |
| alkoxy (OY) | halogen | s/ns C1-C5 alkyl | H | -CN | X of Table 1 |
| alkoxy (OY) | halogen | s/ns C1-C5 alkyl | H | -CONH2 | X of Table 1 |
| alkoxy (OY) | halogen | s/ns C1-C5 alkyl | -CN | H | X of Table 1 |
| alkoxy (OY) | halogen | s/ns C1-C5 alkyl | -CN | -CN | X of Table 1 |
| alkoxy (OY) | halogen | s/ns C1-C5 alkyl | -CN | -CONH2 | X of Table 1 |
| alkoxy (OY) | halogen | s/ns C1-C5 alkyl | -CONH2 | H | X of Table 1 |
| alkoxy (OY) | halogen | s/ns C1-C5 alkyl | -CONH2 | -CN | X of Table 1 |
| alkoxy (OY) | halogen | s/ns C1-C5 alkyl | -CONH2 | -CONH2 | X of Table 1 |
| alkoxy (OY) | alkoxy (OY) | H | H | H | X of Table 1 |
| alkoxy (OY) | alkoxy (OY) | H | H | -CN | X of Table 1 |
| alkoxy (OY) | alkoxy (OY) | H | H | -CONH2 | X of Table 1 |
| alkoxy (OY) | alkoxy (OY) | H | -CN | H | X of Table 1 |
| alkoxy (OY) | alkoxy (OY) | H | -CN | -CN | X of Table 1 |
| alkoxy (OY) | alkoxy (OY) | H | -CN | -CONH2 | X of Table 1 |
| alkoxy (OY) | alkoxy (OY) | H | -CONH2 | H | X of Table 1 |
| alkoxy (OY) | alkoxy (OY) | H | -CONH2 | -CN | X of Table 1 |
| alkoxy (OY) | alkoxy (OY) | H | -CONH2 | -CONH2 | X of Table 1 |
| alkoxy (OY) | alkoxy (OY) | halogen | H | H | X of Table 1 |
| alkoxy (OY) | alkoxy (OY) | halogen | H | -CN | X of Table 1 |
| alkoxy (OY) | alkoxy (OY) | halogen | H | -CONH2 | X of Table 1 |
| alkoxy (OY) | alkoxy (OY) | halogen | -CN | H | X of Table 1 |
| alkoxy (OY) | alkoxy (OY) | halogen | -CN | -CN | X of Table 1 |
| alkoxy (OY) | alkoxy (OY) | halogen | -CN | -CONH2 | X of Table 1 |
| alkoxy (OY) | alkoxy (OY) | halogen | -CONH2 | H | X of Table 1 |
| alkoxy (OY) | alkoxy (OY) | halogen | -CONH2 | -CN | X of Table 1 |
| alkoxy (OY) | alkoxy (OY) | halogen | -CONH2 | -CONH2 | X of Table 1 |
| alkoxy (OY) | alkoxy (OY) | alkoxy (OY) | H | H | X of Table 1 |
| alkoxy (OY) | alkoxy (OY) | alkoxy (OY) | H | -CN | X of Table 1 |
| alkoxy (OY) | alkoxy (OY) | alkoxy (OY) | H | -CONH2 | X of Table 1 |
| alkoxy (OY) | alkoxy (OY) | alkoxy (OY) | -CN | H | X of Table 1 |
| alkoxy (OY) | alkoxy (OY) | alkoxy (OY) | -CN | -CN | X of Table 1 |
| alkoxy (OY) | alkoxy (OY) | alkoxy (OY) | -CN | -CONH2 | X of Table 1 |
| alkoxy (OY) | alkoxy (OY) | alkoxy (OY) | -CONH2 | H | X of Table 1 |
| alkoxy (OY) | alkoxy (OY) | alkoxy (OY) | -CONH2 | -CN | X of Table 1 |
| alkoxy (OY) | alkoxy (OY) | alkoxy (OY) | -CONH2 | -CONH2 | X of Table 1 |
| alkoxy (OY) | alkoxy (OY) | s/ns C1-C5 alkyl | H | H | X of Table 1 |
| alkoxy (OY) | alkoxy (OY) | s/ns C1-C5 alkyl | H | -CN | X of Table 1 |
| alkoxy (OY) | alkoxy (OY) | s/ns C1-C5 alkyl | H | -CONH2 | X of Table 1 |
| alkoxy (OY) | alkoxy (OY) | s/ns C1-C5 alkyl | -CN | H | X of Table 1 |
| alkoxy (OY) | alkoxy (OY) | s/ns C1-C5 alkyl | -CN | -CN | X of Table 1 |
| alkoxy (OY) | alkoxy (OY) | s/ns C1-C5 alkyl | -CN | -CONH2 | X of Table 1 |
| alkoxy (OY) | alkoxy (OY) | s/ns C1-C5 alkyl | -CONH2 | H | X of Table 1 |
| alkoxy (OY) | alkoxy (OY) | s/ns C1-C5 alkyl | -CONH2 | -CN | X of Table 1 |
| alkoxy (OY) | alkoxy (OY) | s/ns C1-C5 alkyl | -CONH2 | -CONH2 | X of Table 1 |

In some embodiments of the present invention the insulin secretagogue of Formula I is selected from the group consisting of the compounds listed in Table 3.

**Table 3**

| **Compound No.** | **Name** |
|---|---|
| 1 | 4-(3-aminophenyl)-7-fluoro-quinoline-2-carbonitrile |
| 2 | 5-[(8-bromo-2-cyano-4-quinolyl)amino]-2,4-dichloro-N,N-dimethyl-benzamide |
| 3 | 8-bromo-4-(2,4-dichloro-5-methoxy-anilino)quinoline-2-carbonitrile |
| 4 | 4-[4-chloro-2-(trifluoromethyl)anilino]-7-fluoro-8-methoxy-quinoline-2-carbonitrile |
| 5 | 4-[4-chloro-2-(trifluoromethyl)anilino]-6-fluoro-quinoline-2-carbonitrile |
| 6 | N-[3-(2-cyano-7-fluoro-4-quinolyl)phenyl]-2,6-difluoro-benzenesulfonamide |
| 7 | 4-[4-chloro-2-(trifluoromethyl)anilino]-8-fluoro-quinoline-2-carbonitrile |
| 8 | 4-(2,6-dichloroanilino)-7-fluoro-8-methoxy-quinoline-2-carbonitrile |
| 9 | N-[4-[[7-(3-aminopropoxy)-6-methoxy-4-quinolyl]oxy]-3-fluoro-phenyl]-3-phenyl-benzenesulfonamide |
| 10 | 4-(2,4-dichloro-5-methoxy-anilino)-6-fluoro-quinoline-2-carbonitrile |
| 11 | N-[3-(2-cyano-7-fluoro-4-quinolyl)phenyl]-3-(trifluoromethyl)benzamide |
| 12 | 4-(2,4-dichloro-5-methoxy-anilino)-7-fluoro-quinoline-2-carbonitrile |
| 13 | 4-[4-chloro-2-(trifluoromethyl)anilino]-7-fluoro-6-methoxy-quinoline-2-carbonitrile |
| 14 | 6,8-dichloro-4-(3-chloro-4-fluoro-anilino)quinoline-2-carbonitrile |
| 15 | 4-(2,4-dichloro-5-methoxy-anilino)-6-methoxy-quinoline-2-carbonitrile |
| 16 | 4-(3,4-dimethylanilino)-7-fluoro-8-methoxy-quinoline-2-carbonitrile |
| 17 | 7-(3-aminopropoxy)-4-(2,4-dichloro-5-methoxy-anilino)-6-methoxy-quinoline-3-carbonitrile |
| 18 | 4-(2,6-dichloroanilino)-8-fluoro-quinoline-2-carbonitrile |
| 19 | 4-(2,4-dichloro-5-methoxy-anilino)-8-fluoro-quinoline-2-carbonitrile |
| 20 | 7-(3-aminopropoxy)-4-(2,4-dichloroanilino)-6-methoxy-quinoline-3-carbonitrile |
| 21 | 4-(2,6-dichloroanilino)-7-fluoro-6-methoxy-quinoline-2-carbonitrile |
| 22 | 4-(2,4-dichloro-5-methoxy-anilino)-8-(trifluoromethyl)quinoline-2-carbonitrile |
| 23 | 4-[2,4-dichloro-5-(dimethylaminomethyl)anilino]-7-fluoro-quinoline-2-carbonitrile |
| 24 | 4-[3-(6,7-dimethoxy-4-quinolyl)anilino]-8-fluoro-quinoline-2-carbonitrile |
| 25 | 2,4-dichloro-5-[(2-cyano-8-fluoro-4-quinolyl)amino]-N,N-dimethyl-benzamide |
| 26 | N-[3-fluoro-4-[[6-methoxy-7-(3-morpholinopropoxy)-4-quinolyl]oxy]phenyl]-3-phenyl-benzenesulfonamide |
| 27 | 4-[3-(6,7-dimethoxy-4-quinolyl)anilino]-8-fluoro-quinoline-2-carboxamide |
| 28 | 2,4-dichloro-5-[(2-cyano-7-fluoro-4-quinolyl)amino]-N,N-dimethyl-benzamide |
| 29 | 4-(2,6-dichloroanilino)-6-fluoro-quinoline-2-carbonitrile |
| 30 | 4-(3,4-dimethylanilino)-8-fluoro-quinoline-2-carbonitrile |
| 31 | 4-(2,4-dichloroanilino)-6,7-dimethoxy-quinoline-3-carbonitrile |
| 32 | 4-(2,4-dichloroanilino)-7-[3-(dimethylamino)propoxy]-6-methoxy-quinoline-3-carbonitrile |
| 33 | 4-(2,4-dichloro-5-methoxy-anilino)-7-fluoro-8-methoxy-quinoline-2-carbonitrile |
| 34 | 4-[2,4-dichloro-5-(dimethylaminomethyl)anilino]-7-fluoro-quinoline-2-carboxamide |
| 35 | 4-(2,4-dichloro-5-methoxy-anilino)-6-methoxy-quinoline-2-carboxamide |
| 36 | N-[4-[[7-[3-(dimethylamino)propoxy]-6-methoxy-4-quinolyl]oxy]-3-fluorophenyl]-3-phenyl-benzenesulfonamide |
| 37 | 4-(2,4-dichloro-5-methoxy-anilino)-6-(3-morpholinopropoxy)quinoline-2-carboxamide |
| 38 | 6-(3-aminopropoxy)-4-(2,4-dichloro-5-methoxy-anilino)-7-fluoro-quinoline-2-carbonitrile |
| 39 | 6,8-dichloro-4-[2,4-dichloro-5-(dimethylcarbamoyl)anilino]quinoline-2-carboxamide |
| 40 | 4-(2,3-dihydro-1,4-benzodioxin-6-ylamino)-8-fluoro-quinoline-2-carbonitrile |
| 41 | N-[[4-(2,4-dichloro-5-methoxy-anilino)-6-fluoro-2-quinolyl]methyl]methanesulfonamide |
| 42 | 4-(2,4-dichloro-5-methoxy-anilino)-6-methoxy-quinoline-2-carboxylic acid |
| 43 | 4-[3-(6,7-dimethoxy-4-quinolyl)anilino]-8-[3-(1,3-dioxoisoindolin-2-yl)propoxy]-7-fluoro-quinoline-2-carbonitrile |
| 44 | 4-(2,4-dichloro-5-methoxy-anilino)-6-fluoro-quinoline-2-carboxamide |
| 45 | 4-(2,3-dihydro-1,4-benzodioxin-6-ylamino)-7-fluoro-6-methoxy-quinoline-2-carbonitrile |
| 46 | N-[3-[[3-cyano-4-(2,4-dichloroanilino)-6-methoxy-7-quinolyl]oxy]propyl]formamide |
| 47 | 8-bromo-4-(2,4-dichloro-5-methoxy-anilino)quinoline-2-carboxamide |
| 48 | 4-(3-chloro-4-fluoro-anilino)-8-fluoro-quinoline-2-carbonitrile |
| 49 | 8-bromo-4-[2,4-dichloro-5-(dimethylcarbamoyl)anilino]quinoline-2-carboxamide |
| 50 | N-[3-[2,4-dichloro-5-[(2-cyano-7-fluoro-4-quinolyl)amino]phenoxy]propyl]acetamide |
| 51 | 4-[5-(3-aminopropoxy)-2,4-dichloro-anilino]-7-fluoro-quinoline-2-carbonitrile |
| 52 | N-[3-[[2-cyano-4-(2,4-dichloro-5-methoxy-anilino)-7-fluoro-6-quinolyl]oxy]propyl]acetamide |
| 53 | 6-fluoro-4-(4-pyridylamino)quinoline-2-carbonitrile |
| 54 | 4-(3,4-dimethylanilino)-7-fluoro-6-methoxy-quinoline-2-carbonitrile |
| 55 | 1-[3-(2-cyano-7-fluoro-4-quinolyl)phenyl]-3-(m-tolyl)urea |
| 56 | 4-(2,4-dichloro-5-hydroxy-anilino)-6-fluoro-quinoline-2-carboxamide |
| 57 | N-(2,4-dichloro-5-methoxy-phenyl)-6-methoxy-7-(3-pyrrolidin-1-ylpropoxy)quinolin-4-amine |
| 58 | 8-fluoro-4-[4-[(4-methylpiperazin-1-yl)methyl]anilino]quinoline-2-carbonitrile |
| 59 | 4-(2,4-dichloro-5-hydroxy-anilino)-6-fluoro-quinoline-2-carboxamide |
| 60 | 7-(4-aminobutoxy)-4-(2,4-dichloro-5-methoxy-anilino)quinoline-2-carbonitrile |
| 61 | 4-[2,4-dichloro-5-[3-(1,3-dioxoisoindolin-2-yl)propoxy]anilino]-7-fluoro-6-methoxy-quinoline-2-carbonitrile |
| 62 | 4-(2,4-dichloro-5-methoxy-anilino)-7-(3-hydroxypropoxy)quinoline-2-carboxamide |
| 63 | 4-(2,4-dichloro-5-methoxy-anilino)-6-[3-(1,3-dioxoisoindolin-2-yl)propoxy]-7-fluoro-quinoline-2-carbonitrile |
| 64 | 2-(aminomethyl)-N-(2,4-dichloro-5-methoxy-phenyl)-7-fluoro-quinolin-4-amine |
| 65 | 8-bromo-4-(2,4-dichloro-5-methoxy-anilino)-6-fluoro-quinoline-2-carbonitrile |
| 66 | 4-[(2,6-dichloro-4-pyridyl)amino]-7-fluoro-8-methoxy-quinoline-2-carbonitrile |
| 67 | 1-[3-(2-cyano-7-fluoro-4-quinolyl)phenyl]-3-(2,4-difluorophenyl)urea |
| 68 | tert-butyl N-[3-[[4-[2-fluoro-4-[(3-phenylphenyl)sulfonylamino]phenoxy]-6-methoxy-7-quinolyl]oxy]propyl]carbamate |
| 69 | 2-[3-[[4-[3-[(2-cyano-8-fluoro-4-quinolyl)amino]phenyl]-6-methoxy-7-quinolyl]oxy]propylcarbamoyl]benzoic acid |
| 70 | N-[3-(2-cyano-7-fluoro-4-quinolyl)phenyl]-3-methoxy-benzamide |
| 71 | N-[4-chloro-2-(trifluoromethyl)phenyl]-4-[(2-cyano-7-fluoro-6-methoxy-4-quinolyl)oxy]benzenesulfonamide |
| 72 | 1-[3-(2-cyano-7-fluoro-4-quinolyl)phenyl]-3-[3-(trifluoromethyl)phenyl]urea |
| 73 | 7-benzyloxy-N-(2,4-dichloro-5-methoxy-phenyl)-6-methoxy-quinolin-4-amine |
| 74 | 4-(2,4-dichloro-5-methoxy-anilino)-7-fluoro-6-methoxy-quinoline-3-carbonitrile |
| 75 | 4-(2,4-dichloro-5-methoxy-anilino)-7-fluoro-6-methoxy-quinoline-2-carbonitrile |
| 76 | N-(2,4-dichloro-5-methoxy-phenyl)-2-methyl-6-(3-morpholinopropoxy)quinolin-4-amine |
| 77 | 2-(aminomethyl)-N-(2,4-dichloro-5-methoxy-phenyl)-6-fluoro-quinolin-4-amine |
| 78 | N-(2,4-dichloro-5-methoxy-phenyl)-6-methoxy-2-methyl-quinolin-4-amine |
| 79 | N-[[4-(2,4-dichloro-5-methoxy-anilino)-6-fluoro-2-quinolyl]methyl]acetamide |
| 80 | N-(2,4-dichloro-5-methoxy-phenyl)-6-methoxy-7-[3-(4-methylpiperazin-1-yl)propoxy]quinolin-4-amine |

Particularly preferred are compounds 1 to 30, 1 to 20, 1 to 15, 2, 3, 4, 5, 6, 7, 8, 9, 10, 11, 12, 13, 14 or 75. Most preferred are compounds 1, 2, 3, 4, 5 and 75.

The present insulin secretagogue of Formula I is able to induce insulin secretion in pancreatic beta cells. Insulin secretion can be measured by standard methods known to the person skilled in the art, such as the method described in more detail in Example 1 of the present application. The ability to induce insulin secretion can be assessed in vitro. For this purpose different primary human, mouse, rat or rabbit pancreatic beta cells can be used. Also, established beta cell lines known to the person skilled in the art can be used. For example, RIN-5AH, TC-6, BRIN-BD11, and INS-1E can be used.

In some embodiments the insulin secretagogue of Formula I is able to induce divalent calcium cation influx in pancreatic beta cells. Divalent calcium cation influx can be measured by standard methods known to the person skilled in the art, such as the methods described in more detail in the examples of the present application. The ability to induce divalent calcium cation influx in pancreatic beta cells can be assessed in vitro. For this purpose different primary human, mouse, rat or rabbit pancreatic beta cells can be used. Also, established beta cell lines known to the person skilled in the art can be used. For example, RIN-5AH, TC-6, BRIN-BD11, and INS-1E can be used. Most preferred are RIN-5AH cells.

In some embodiments the insulin secretagogue of Formula I is able to activate channels for divalent calcium cations in pancreatic beta cells. Activation of channels for divalent calcium cations can be measured by standard methods known to the person skilled in the art, such as the methods described in more detail in the examples of the present application. The ability to activate channels for divalent calcium cations in pancreatic beta cells can be assessed in vitro. For this purpose different primary human, mouse, rat or rabbit pancreatic beta cells can be used. Also, established beta cell lines known to the person skilled in the art can be used. For example, RIN-5AH, TC-6, BRIN-BD11, and INS-1E can be used. Most preferred are RIN-5AH cells. The channels for divalent calcium cations can be of the L-, T-, N- and P/Q type.

In one aspect the present invention relates to the insulin secretagogue of Formula I for use in a therapeutic method. A therapeutic method means the treatment of a disease with the insulin secretagogue of Formula I. For this purpose, the insulin secretagogue of Formula I may be formulated as a pharmaceutical composition comprising the insulin secretagogue of Formula I and additional pharmaceutical excipients known to the person skilled in the art, such as diluents fillers, binders, disintegrants, lubricants, coloring agents and preservatives. Such a pharmaceutical composition may be formulated, for example, as a tablet or as a capsule for sustained or immediate release.

In this regard, in one aspect the present invention relates to a pharmaceutical composition comprising the insulin secretagogue of Formula I. The pharmaceutical composition may further include additional pharmaceutical excipients known to the person skilled in the art, such as diluents fillers, binders, disintegrants, lubricants, coloring agents and preservatives. Such a pharmaceutical composition may be formulated, for example, as a tablet or as a capsule for sustained or immediate release.

In another aspect the present invention relates to the insulin secretagogue of Formula I for use in the treatment and/or prevention of diabetes. In the context of the present application, the term diabetes is used interchangeably with the term diabetes mellitus. In the context of the present application diabetes refers to the disease diabetes mellitus in all its forms known to the person skilled in the art. In the context of the present application this includes type I diabetes, type II diabetes, gestational diabetes mellitus (GDM), pre-diabetes, latent autoimmune diabetes of adults (LADA), congenital diabetes, obesity-related diabetes, cystic fibrosis-related diabetes steroid diabetes induced by high doses of glucocorticoids, maturity onset diabetes of the young (MODY), intermediate hyperglycemia, and any other form of diabetes that is characterized by abnormally elevated blood glucose levels, due to a reduced, or lack of, functional insulin production by the pancreatic beta cells and/or reduced sensitivity to insulin. Correspondingly, in one aspect the present invention relates to a pharmaceutical composition comprising the insulin secretagogue of Formula I for use in the treatment and/or prevention of diabetes.

Methods for the diagnosis of diabetes, and its various subtypes listed above, are known to the person skilled in the art. Diabetes in all its forms is characterized by a recurrent or elevated blood glucose level. Diabetes may be characterized by a fasting plasma glucose level of greater or equal to 6.1, 6.9 or 7.0 mmol per liter or a plasma glucose level of greater or equal to 7.8 or 11.1 mmol per liter two hours after oral challenge with 75 g of glucose (glucose tolerance test). Alternatively, or in addition, diabetes may be characterized by glycated hemoglobin (hemoglobin A1c, HbA1c, A1C, or Hb1c; sometimes also HbA1c or HGBA1C). In particular, a level of glycated hemoglobin greater or equal to 48 mmol/mol may be characteristic for diabetes.

In one embodiment the invention relates to the treatment of all forms of diabetes except type I diabetes. Type I diabetes is characterized by the loss of pancreatic beta cells and therefore the loss of the ability to produce insulin. In any other type of diabetes, beta cells are still present and may be stimulated pharmacologically by the insulin secretagogue of Formula I to secrete insulin. The insulin secretagogue of Formula I can be used to compensate for a lack of sufficient insulin secretion by pancreatic beta cells or can be used to overcome a reduced sensitivity to insulin by increasing the amount of secreted insulin.

In one embodiment of the invention the insulin secretagogue of Formula I can be for use in humans or other mammals, in particular, cats, dogs, sheep, swine, or cow.

In one embodiment the invention relates to the insulin secretagogue of Formula I for use in the treatment of diabetes when the use of sulfonylureas, meglitinides, or biguanides is contraindicated. The use of sulfonylureas or biguanides may be contraindicated in patients with sulfa allergies. The use of meglitinides may be contraindicated in patients with renal insufficiency.

In yet another aspect, the invention relates to the use of the present insulin secretagogues for inducing insulin secretion in pancreatic beta cells in vitro, the use of the present insulin secretagogues for inducing divalent calcium cation influx in pancreatic beta cells in vitro, or the use of the present insulin secretagogues for activating voltage-dependent calcium channels in pancreatic beta cells in vitro. Preferably the insulin secretagogue of Formula I can be used to induce insulin secretion or calcium cation influx in human, murine or rat cells, for example, in RIN-5AH, TC-6, BRIN-BD11, and INS-1 E cells, most preferably in RIN-5AH cells. Preferably the insulin secretagogue of Formula I can be used activating voltage-dependent calcium channels of the L-, T-, N- and P/Q type, more preferably of the L- and T-type, most preferably of the T-type.

The term "insulin secretagogue" refers to a small organic molecule, such as a molecule with a molecular weight of less than 900 Dalton, preferably, less than 800, 700, 600, or 500 Dalton and comprising carbon atoms that is able to directly induce insulin secretion in pancreatic beta cells. Insulin secretion can be measured by methods known to the person skilled in the art as exemplified in the examples of the present application.

The terms "directly stimulates insulin secretion" or "directly induces insulin secretion" as used herein refers to a compound's ability to induce, stimulate, promote or cause insulin secretion in pancreatic beta cells when the compound is contacted with pancreatic beta cells, for example, when pancreatic beta cells are grown in tissue culture and the compound is added to the growth medium of the pancreatic beta cells. A compound's ability to directly induce, stimulate, promote or cause insulin secretion in pancreatic beta cells can be assessed by the person skilled in the art by using methods known in the art, such as the methods described in detail in the Examples provided with this application.

The term "halogen" as used herein refers to fluorine, chlorine, bromine, and iodine. The most preferred halogens are fluorine, chlorine and bromine.

The term "primary, secondary or tertiary amide" as used herein refers to substitutions of the type, -CONH₂, -CONHR and -CONR₁R₂, respectively. In the context of the term "primary, secondary or tertiary amid", R, R₁ and R₂ independently refer to, for example, a methyl, ethyl, n-propyl or iso-propyl.

The term "primary, secondary or tertiary alkylamine" as used herein preferably refers to methyl, ethyl, propyl, butyl or pentyl substituents with a terminal -NH, -NHR₁, or -NR₁R₂ group. In the context of the term "primary, secondary or tertiary alkylamine" R₁ and R₂ independently refer to, for example, a methyl, ethyl, n-propyl or iso-propyl.

The term "alkoxy" as used herein refers to the group -O-R, wherein R is a substituted or non-substituted C1 to C5 alkyl. Examples of alkoxy groups are methoxy, ethoxy, propoxy, butoxy. The alkoxy group may be substituted, for example, with halogens, oxygen-, sulfur- or nitrogen-containing functional groups, heterocycles or aryl groups. In particular, the alkoxy group may be terminally modified with primary amide group, a primary amine, 1-pyrrolidin-yl, or phthalimide. Preferably the alkoxy group is selected from the group consisting of aminopropoxy, N,N-dimethyl-3-aminopropoxy, 3-(morpholin-4-yl)propoxy, 3-hydroxypropoxy, 3-(pyrrolidin-1-yl)propoxy, 3-acetamidopropoxy, 3-carbamidopropoxy, 3-formamidopropoxy, N-3-phthalimidopropoxy, 4-aminobutoxy, N,N-dimethyl-4-aminobutoxy, 4-(morpholin-4-yl)butoxy, 4-hydroxybutoxy, 4-(pyrrolidin-1-yl)butoxy, 4-acetamidobutoxy, 4-carbamidobutoxy, 4-formamidobutoxy and N-4-phthalimidobutoxy.

The term "alkyl" as used herein refers to a straight-chain or branched saturated aliphatic hydrocarbon with 1 to 20 (C1-C20), preferably 1 to 15 (C1-C15), more preferably 1 to 10 (C1-C10), and most preferably 1 to 5 (C1-C5) carbon atoms. For example, "alkyl" may refer to methyl, ethyl, n-propyl, isopropyl, n-butyl, s-butyl, isobutyl, t-butyl, n-pentyl, 1-methylbutyl, 2-methylbutyl, 3-methylbutyl, 1,1-dimethylpropyl, 1-methyl-2-methylpropyl, 2,2-dimethylpropyl, and 1-ethylpropyl.

The "alkyl" group may be substituted with, for example, halogens, such as fluorine, chlorine and bromine, amines, such as a primary amine (-NH₂), primary amide, hydroxyl (-OH), further oxygen-, sulfur- or nitrogen-containing functional groups, heterocycles, or aryl substituents, such as phenyl and napthyl.

The term "aryl" as used herein refers to aromatic polycarbocyclic substituents, such as phenyl and naphtyl. The aryl substituent may be mono-, di- or trisubstituted, with another aryl, such as phenyl, halogen, trifluoromethyl, pentafluoroentyl or alkoxy, such as methoxy, ethoxy or propoxy.

The term "alkylaryl" as used herein preferably refers to an ethyl, methyl or n-propyl with a terminal aryl substituent.

The term "heterocycle" as used herein refers to, for example, a aromatic or saturated five or six-membered monocyclic carbocycle with one or more oxygen-, nitrogen- or sulfur-atoms. For example, "heterocycle" refers to pyrrolidine, piperidine, piperazine, morpholine, furan, thiophene, pyrrole, imidazole, oxazole, pyridine, pyrimidine and pyrazine. The term "heterocycle" as used herein also refers to, for example, bicyclic aromatic or saturated 11- or 12-membered carbocycles with one or more oxygen-, nitrogen- or sulfur-atoms. For example, "heterocycle" refers to quinoline, isoquinoline, quinazoline, indole, isoindole, indazole, and purine. The heterocycle may be substituted with, for example, methyl, ethyl, propyl, methoxy, ethoxy or propoxy

The term "alkylheterocycle" as used herein preferably refers to an ethyl, methyl or n-propyl with a terminal heterocycle substituent.

### EXAMPLES

### Synthetic Example

The following exemplarily illustrates the synthesis of the insulin secretagogues according to the present invention.

Insulin secretagogues of the present invention with R₄ being nitrile, R₅ being hydrogen and X being a substituted N-phenyl were synthesized according to the following synthetic scheme:

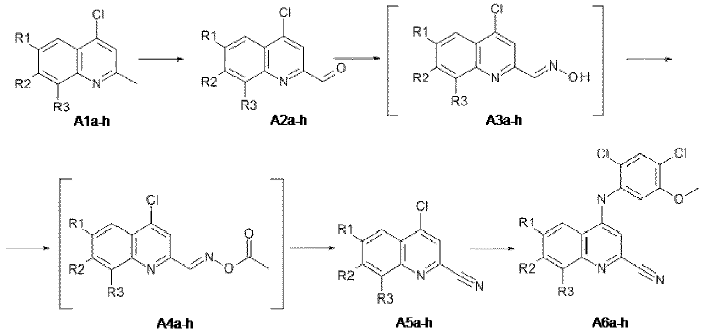

Compounds A1 to A6 have the following substituents R₁, R₂ and R₃:

**Table 4:**

| | **R₁** | **R₂** | **R₃** |
|---|---|---|---|
| a | OCH3 | F | H |
| b | OCH3 | H | H |
| c | H | F | H |
| d | F | H | H |
| e | H | H | F |
| f | H | H | Br |
| g | H | H | CF3 |
| h | H | F | OCH3 |

### 4-Chloro-7-fluoro-6-methoxy-quinoline-2-carbaldehyde (A2a):

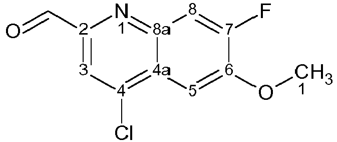

The mixture of 9.03g (40 mmol) 4-chloro-7-fluoro-6-methoxy-2-methylquinoline, 4,44g selendioxide, 90 ml dioxane was refluxed for 2 hours. The hot mixture was filtered through celite, washed with hot dioxane, and then the filtrate was evaporated in vacuum. The residue was taken up in the mixture of 400 ml of ethylacetate and 100 ml of sodium hydrogencarbonate aqueous solution. The mixture was filtered and then the two layers of the filtrate were separated. The aqueous solution was extracted two times with ethyl acetate, the organic layers were combined, washed with brine, dried over sodium sulfate, filtered and evaporated. The residue was solidified under diisopropyl ether. Thus 5.65 g of title compound was obtained. Yield: 59 %. LCMS 97 %, MW calc: 239.0149, MH+ 240.0/ MH- 238.0 /C11H7CIFNO2

### 4-Chloro-6-methoxy-quinoline-2-carbaldehyde (A2b):

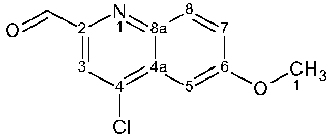

Compound A2b was prepared as it was described for A2a, starting from 8.31 g (40 mmol) 4-chloro-6-methoxy-2-methylquinoline resulted 5.58 g title compound. Yield: 63% MW calc: 221.0244, MH+ 222.03/ MH-220.02 /C11H8CINO2

### 4-Chloro-7-fluoro-quinoline-2-carbaldehyde (A2c):

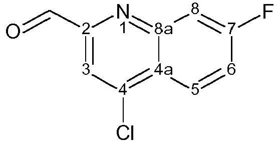

The process was the same as described in example A2a, but 7.82 g (40 mmol) of 4-chloro-7-fluoro-2-methylquinoline was used as starting material. Thus 4.87 g of title compound was obtained. Yield: 58 %. LCMS 96 % MW calc: 209.0044, MH+ 210.01/ MH-208.0 /C10H5CIFNO

### 4-Chloro-6-fluoro-quinoline-2-carbaldehyde (A2d):

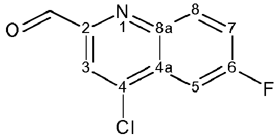

The process was the same as described in example A2a, but 7.82 g (40 mmol) of 4-chloro-6-fluoro-2-methylquinoline was used as starting material. Thus 5.76 g of title compound was obtained. Yield: 52 %. LCMS 99% MW calc: 209.0044, MH+ 210.01/ MH-208.0 /C10H5CIFNO

### 4-Chloro-8-fluoro-quinoline-2-carbaldehyde (A2e):

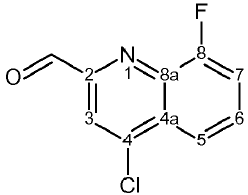

The process was the same as described in example A2a, but 7.82 g (40 mmol) of 4-chloro-8-fluoro-2-methylquinoline was used as starting material. Thus 4.33 g of title compound was obtained. Yield: 58 %. LCMS 96 % MW calc: 209.0044, MH+ 210.01/ MH-208.0 /C10H5CIFNO

### 8-Bromo-4-chloro-quinoline-2-carbaldehyde (A2f):

The process was the same as described in example A2a, but 10.24 g (40 mmol) of 4-chloro-8-bromo-2-methylquinoline was used as starting material. Thus 5.52 g of title compound was obtained. Yield: 51 %. LCMS 98 % MW calc: 268.9243, MH+ 269.93/ MH-267.92/ C₁₀H₅BrCINO

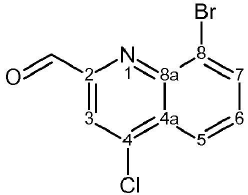

### 4-Chloro-8-trifluoromethyl-quinoline-2-carbaldehyde (A2g):

The process was the same as described in example A2a, but 10.24 g (40 mmol) of 4-chloro-8-trifluoromethyl-2-methylquinoline was used as starting material. Thus 5.52 g of title compound was obtained. Yield: 45 %. LCMS 98 % MW calc: 259.0012, MH+ 260.01/ MH-257.99/ C₁₁H₅CIF₃NO

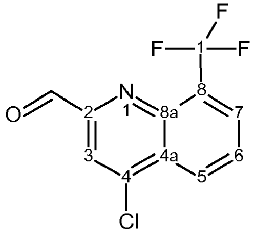

### 4-Chloro-7-fluoro-8-methoxy-quinoline-2-carbaldehyde (A2h):

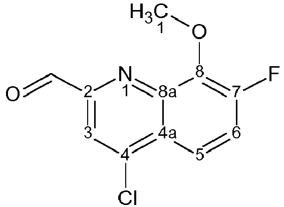

The process was the same as described in example A2a, but 9.02 g (40 mmol) of 4-chloro-7-fluoro-8-methoxy-2-methylquinoline was measured as starting material. Thus 7.25 g of title compound was obtained. Yield: 75 %. LCMS 94 % MWcalc. 239.0149/ MH+ 240.02/MH-238.01 /C₁₁H₇CIFNO₂

### 4-Chloro-7-fluoro-6-methoxy-quinoline-2-carbonitrile (A5a):

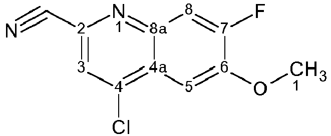

The mixture of 4.78 g (20 mmol) of 4-chloro-7-fluoro-6-methoxy-quinoline-2-carbaldehyde (A2a), 1.40 g (20 mmol) hydroxylamine hydrochloride and 24 ml of dimethyl formamide was stirred at a temperature of 50 grad Celsius for 3 hours. 20 ml of acetic anhydryde was added to this mixture, and it was stirred at 80 grad Celsius for overnight. The mixture was evaporated in vacuo, the residue was taken up in the mixture of 100 ml of ethyl acetate and 50 ml of water. The mixture was filtered then the two layers of the filtrate were separated. The aqueous solution was extracted two times with ethyl acetate, the organic layers were combined, washed with brine, dried over sodium sulfate, filtered and evaporated. The residue was purified by column chromatography (eluent: hexane - ethyl acetate in a 7:3 mixture). The pure product solidified under diisopropyl ether.

Thus 2.15 g of title compound was obtained. Yield: 45 %. LCMS 97 % MWcalc. 236.0153/ MH+ 237.02/MH-235.01 / C₁₁H₆CIFN₂O

### 4-Chloro-6-methoxy-quinoline-2-carbonitrile (A5b):

The title compound was synthesized according to the process described for A5a, except that 4.43 g (20 mmol) 4-chloro-6-methoxy-quinoline-2-carbaldehyde was used as starting material. 1.66 g title compound was isolated. Yield: 38%. LCMS 98% MWcalc. 218.0247/ MH+ 217.02/MH-219.03 / CₙH₇CIN₂O

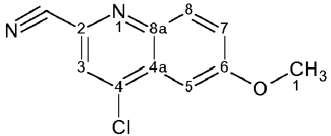

### 4-Chloro-7-fluoro-quinoline-2-carbonitrile (A5c):

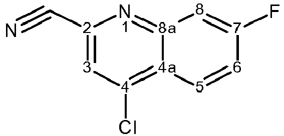

The process was similar as described in example A5a, but 4.19 g (20 mmol) of 4-chloro-7-fluoro-quinoline-2-carbaldehyde (A2c) was used as starting material. Thus 1.61 g of title compound was obtained. Yield: 39 %. LCMS 97 % MWcalc. 206.0047/ MH+ 206.99/ MH-205.01 /C₁₀H₄CIFN₂

### 4-Chloro-6-fluoro-quinoline-2-carbonitrile (A5d):

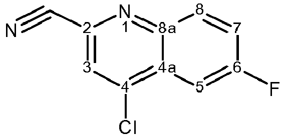

The process was similar as described in example A5a, but 4.19 g (20 mmol) of 4-chloro-6-fluoro-quinoline-2-carbaldehyde (A2d) was used as starting material. Thus 1.40 g of title compound was obtained. Yield: 34 %. LCMS 98 % MWcalc. 206.0047/ MH+ 207.01/ MH-205.00 /C₁₀H₄CIFN₂

### 4-Chloro-8-fluoro-quinoline-2-carbonitrile (A5e):

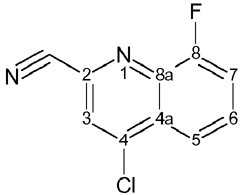

The process was similar as described in example A5a, but 4.19 g (20 mmol) of 4-chloro-8-fluoro-quinoline-2-carbaldehyde (A2e) was used as starting material. Thus 1.21 g of title compound was obtained. Yield: 29 %. LCMS 97 % MWcalc. 206.0047/ MH+ 207.02/ MH-205.01/C₁₀H₄CIFN₂

### 8-Bromo-4-chloro-quinoline-2-carbonitrile (A5f):

The process was similar as described in example A5a, but 5.41 g (20 mmol) of 4-chloro-8-bromo-quinoline-2-carbaldehyde (A2f) was used as starting material. Thus 1.66 g of title compound was obtained. Yield: 31 %. LCMS 97 % MWcalc. 265.9247/ MH+ 267.92/ MH-264.93 /C₁₀H₄BrCIN₂

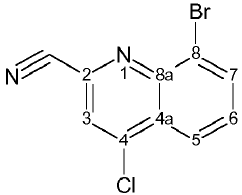

### 4-Chloro-8-trifluoromethyl-quinoline-2-carbonitrile (A5g):

The process was similar as described in example A5a, but 5.19 g (20 mmol) of 4-chloro-8-trifluoromethyl-quinoline-2-carbaldehyde (A2g) was used as starting material. Thus 1.81 g of title compound was obtained. Yield: 35 %. LCMS 98 % MWcalc. 256.0015/ MH+ 257.01/ MH-254.98/C₁₁H₄CIF₃N₂

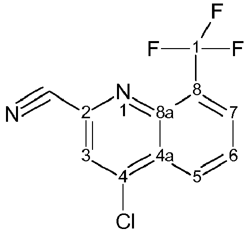

### 4-Chloro-7-fluoro-8-methoxy-quinoline-2-carbonitrile (A5h):

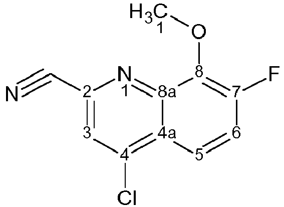

The process was similar described in example A5a, but 4.80 g (20 mmol) of 4-chloro-7-fluoro-8-methoxy-quinoline-2-carbaldehyde (A2h) was measured as starting material. Thus 2.95 g of title compound was obtained. Yield: 62 %. LCMS 98 % MWcalc. 236.0153/ MH+ 237.03/ MH-235.02 /C₁₁H₆CIFN₂O

### Example 1: Quinoline compounds act as insulin secretagogues

Culture medium and supplements were purchased from Life Technologies. The RIN-5AH insulinoma beta cell line was cultured in RPMI 1640 supplemented with 10% FBS, 2 mM glutamine. All culture media contained 1x penicillin-streptomycin. Cells were grown on 15 cm culture plates at 37°C under 5% CO2 in high humidity atmosphere.

To determine the insulin level from the supernatants insulin ELISA kits from Millipore or Mercodia were used. RIN-5AH cells were seeded in 96 well or 12 well plates and grown overnight. RIN-5AH cells never reached more than 80% confluency before treatment. Cells were washed once with PBS before treatment. Cells were treated in medium for 2 hours and with 5 µM of insulin secretagogue according to the present invention, if not otherwise indicated. After treatment 100ul of supernatant was taken. Samples were assayed immediately or stored at -20°C until measurement. Dilutions were made if necessary and ELISA was subsequently performed according to the user's manual. Insulin secretion was normalized to cell number by MTT assay or to total protein amount by BCA assay (ThermoFisher Scientific, 23225). Plates were read on BioTrek Elx800 absorbance reader at 450 nm.

Various quinoline compounds according to the present invention were tested for their ability to induce insulin secretion in beta cells according to the above described protocol. The following list exemplary highlights 29 insulin secretagogues of the present invention that displayed a substantial increase of insulin secretion in beta cells.

**Table 5 (Compound number (No.) refers to the compound number as defined in Table 3, AV_INSULlN refers to the percent increase in insulin secretion over a DMSO control)**

| **Compound No.** | **AV_INSULIN** |
|---|---|
| 1 | 151% |
| 2 | 125% |
| 3 | 116% |
| 4 | 101% |
| 5 | 96% |
| 6 | 94% |
| 7 | 93% |
| 8 | 93% |
| 9 | 90% |
| 10 | 89% |
| 11 | 86% |
| 12 | 85% |
| 13 | 83% |
| 14 | 78% |
| 15 | 78% |
| 16 | 78% |
| 17 | 74% |
| 18 | 73% |
| 19 | 70% |
| 20 | 67% |
| 21 | 65% |
| 22 | 58% |
| 23 | 57% |
| 24 | 57% |
| 25 | 48% |
| 26 | 47% |
| 27 | 46% |
| 28 | 45% |
| 29 | 41% |
| 75 | 116% |

### Example 2: Quinoline compounds of the present invention have improved activity

It has been previously shown that some quinoline compounds are able to induce insulin secretion in beta cells (Gribble at al, 2000, British Journal of Pharmacology, 131, 756; Henquin, 1982, Endocrinology, 110, 1325). Therefore the ability to induce insulin secretion in beta cell was compared between the known quinolines quinidine and chloroquine, which are both actively used pharmaceuticals. Quinidine is a class I antiarrhythmic agent and chloroquine is used in the prevention of malaria. Surprisingly, it has been found that the insulin secretagogues of the present invention have outstandingly superior activity in inducing insulin secretion in beta cells when compared to quinidine or chloroquine.

RIN-5AH beta cells were treated with 5 µM compound 12 or 50 µM quinidine or chloroquine according to the above described protocol (Example 1).

Even at just 10% the dose of quinidine or chloroquine, compound compound 12 displayed a significantly superior activity in inducing insulin secretion in this essay (Figure 1).

### Example 3: Quinoline compounds induce calcium influx in beta cells

It has been surprisingly found that the compounds of the present invention induce divalent calcium cation influx, through voltage dependent calcium channels in pancreatic beta cells. Calcium influx was measured by FACS analysis, similar to protocols previously described (Brewis, I. A., journal of andrology 21, 238-249, doi:10.1002/j.1939-4640.2000.tb02101.x (2000); Vines, A., Cytometry Part A 77A, 1091-1097, doi:10.1002/cyto.a.20974 (2010); Schepers, E., Journal of Immunological Methods 348, 74-82, doi:http://dx.doi.org/10.1016/j.jim.2009.07.002 (2009)).

RIN-5AH cells (30,000 cells per well) were plated in 12 well plates and incubated overnight at 37°C and 5% CO2. Cells were stained with Fluo-4 AM (molecular probes, F14201) by using a 3µM stock solution prepared in HBSS with Ca2+ and Mg2+ (HBSS++) and with addition of a final amount of 0.02% Pluronic-F127 (molecular probes, P6867). Cells were incubated with 600ul of the dye/well for 1h at 37°C, in 5% CO2. They were washed 2x with Ca2+ and Mg2+ free HBSS (HBSS--) (Gibco, 14170) before moving to the next step of the protocol (see below).

Cells were trypsinized for three minutes at 37°C and 5% CO2. The cell suspension was then diluted by adding 1ml HBSS-- / well and centrifuged for 3 minutes at 1200 RPM. Cells were resuspended in 1 mL of HBSS++ or HBSS-- before starting the measurement. Base fluorescent signal was recorded for 30 seconds followed by treatment with the various compounds by quickly adding the compounds to the FACS analysis tubes, while acquisition was not interrupted (gaps). Final DMSO concentration did not exceed 0.1%. Samples were measured continuously for the desired time and all measurements were completed within 40 minutes. Unstained cells and stained cells in external buffer without Ca2+ and Mg2+ (HBSS--) were used as negative controls for the treatments. Data were acquired on BD-FACSCalibur and the visualizations were prepared by FlowJo software.

Following the above protocol compound 12 was investigated by FACS analysis for its ability to induce divalent calcium cation influx in RIN-5AH cells. It was found that compound 12 induces divalent calcium cation influx in RIN-5AH cells (Figure 2).

Further compounds of the present invention were also tested for their ability to induce divalent calcium cation influx in RIN-5AH cells according to the above protocol. Compounds 4, 5, 7, 8, 12, 13, and 18 induced divalent calcium cation influx in RIN-5AH cells (Figure 2).

### Example 4: Patch clamp analysis

For the purpose of investigation of membrane potential changes and membrane depolarization that triggers and activate voltage dependent calcium channels in beta cells, whole cell patch clamp studies were conducted with the compounds of the present invention. RIN-5AH cells were trypsinized and suspended in medium. Cells were transferred into the recording chamber and allowed to settle down before beginning a slow perfusion with the external buffer (138.0 mM; NaCl; 5.6 mM KCl; 11.1 mM Glucose; 1.2 mM; MgCl2; 2.6 mM CaCl2; 10.0 mM HEPES). Cells were sealed and patched by the application of a gentle negative pressure. Holding membrane potential was set up to -70mV for voltage clamp experiments. After patching the cells, at least 10s of base signal was recorded for both voltage clamp and current clamp measurements. External stimulation of cells was done by using Picospritzer II instrument (Parker) with 5psi for 30s with the desired concentrations (50uM and 500uM for compound 12 and 500uM for glibenclamide as control). The total recording time was set to 1-4 minutes.

After applying "puffs" for 30s of the quinoline compound on the cells a shift in membrane potential was registered from -70mV up to -20mV in current clamp mode (CC) (Figures 3A and 3B). An increase in inward current could also be detected in voltage clamp mode (VC), which lasted longer compared to GBA stimulation (Figures 4A and 4B). Similarly to the results detected by FACS, where compound 12 treatment generated a sustained calcium influx (Example 3).

When comparing VC and CC curves for compound 12 treatment, it was visible that the effect in VC is shorter than in CC, thus the compound keeps the membrane potential for a prolonged time in depolarized state (>240 sec) while ion current stops. In contrast to GBA, where membrane potential returned to its initial value after 90 sec. The treatment with compound 12 increased the inward current immediately, unlike GBA where a small delayed response could be observed. Applying the same concentrations of compound 12 and GBA, the developed difference of maximal inward ion current was more than 10 fold in the cells stimulated by compound 12. By comparing AUC values of VC, lower concentration of the drug (50 µM) could generate a comparable rate of inward current as treatment with higher concentration (500 µM) and the maximal inward current reached by treatment was still more than 5 fold more than in GBA treated cells (Figures 4A and 4B). These observations are indicating that the insulin secretagogues of the present invention most likely do not act on ATP-sensitive K+(KATP) channel, as do sulfonyl ureas, such as GBA, but they have a different and quicker mechanism to activate voltage-gated Ca2+ channels in RIN-5AH cells. The AUC of the curves were analyzed in GraphPad Prism Software (Figures 3 to 5).

## Claims

1. An insulin secretagogue of formula I: 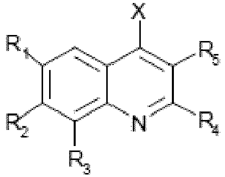 wherein
X is selected from the group consisting of substituted or non-substituted phenyl, N-phenyl, O-phenyl, pyridyl, N-pyridyl, O-pyridyl, pyrimidyl, N-pyrimidyl, and O-pyrimidyl;
**R₁**, **R₂** and **R₃** are independently selected from the group consisting of (a) hydrogen, (b) halogen, (c) substituted or non-substituted C1-C5 alkyl, (d) hydroxyl, and (e) alkoxy (OY), wherein
Y is a substituted or non-substituted C1-C5 alkyl;
**R₄** and **R₅** are independently selected from the group consisting of (a) hydrogen, (b) nitrile, (c) primary, secondary or tertiary amide, (d) carboxylic acid, (e) carboxylic acid ester, (f) methyl-N-methanesulfonamide and (g) alkylamine; and
wherein the insulin secretagogue stimulates insulin secretion in pancreatic beta cells.

2. The insulin secretagogue according to claim 1, wherein R₁, R₂ and R₃ are independently selected from the group consisting of (a) hydrogen, (b) fluorine, chlorine, or bromine, (c) methoxy, ethoxy, propoxy, butoxy, pentoxy, 3-aminopropoxy, N,N-dimethyl-3-aminopropoxy, 3-(morpholin-4-yl)propoxy, 3-hydroxypropoxy, 3-(pyrrolidin-1-yl)propoxy, 3-acetamidopropoxy, 3-formamidopropoxy, N-3-phthalimidopropoxy, 4-aminobutoxy, N,N-dimethyl-4-aminobutoxy, 4-(morpholin-4-yl)butoxy, 4-hydroxybutoxy, 4-(pyrrolidin-1-yl)butoxy, 4-acetamidobutoxy, 4-formamidobutoxy, N-4-phthalimidobutoxy and (g) trifluoromethyl or pentafluoroethyl.

3. The insulin secretagogue according to claim 1 or 2, wherein R₄ and R₅ are independently selected from the group consisting of (a) -CN, (b) -CONH₂, -CONHCH₃, or-CONH(CH₃)₂(c)-COOH, -COOCH₃, or -COO(CH₃)₂ (d) -CH₂NH₂, -(CH₂)₂NH₂, or -(CH₂)₃NH₂ (e) -CH₂NSO₂CH₃ and (f) hydrogen.

4. The insulin secretagogue according to any one of claims 1 to 3, wherein R₄ is -CN and R₅ is hydrogen, R₄ and R₅ are both hydrogen, R₄ is hydrogen and R₅ is -CN, or R₄ is CONH₂ and R₅ is hydrogen.

5. The insulin secretagogue according to any one of claims 1 to 4, wherein X is selected from the group consisting of substituted or non-substituted phenyl, N-phenyl, O law states thought to is that you should tell-phenyl, pyridyl, N-pyridyl, O-pyridyl, pyrimidyl, N-pyrimidyl, or O-pyrimidyl, wherein the phenyl, pyridyl or pyrimidyl is substituted with at least one substituent selected from the group consisting of
(a) halogen
(b) trifluoromethyl or pentafluoroethyl
(c) alkoxy
(e) primary, secondary or tertiary amide,
(f) primary, secondary, or tertiary alkylamine,
(g) substituted or non-substituted C1-C5 alkyl
(i) aryl (Ar), alkylaryl, heterocycle (Het), or alkylheterocycle,
(j) amine, and
(k) -NSO₂Ar, -NCOAr, -NCONAr, or -SO₂NAr.

6. The insulin secretagogue according to any one of claims 1 to 5, wherein X is selected from the group consisting of phenyl, O-phenyl, and N-phenyl.

7. The insulin secretagogue according to any one of claims 1 to 6, wherein X is selected from the group consisting of:

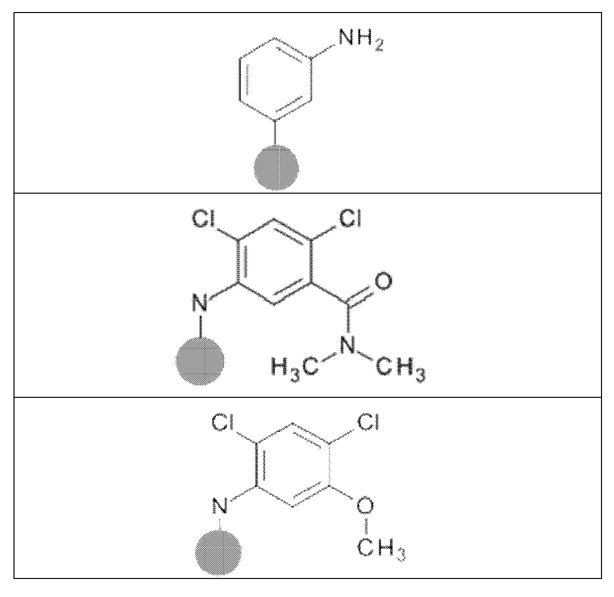

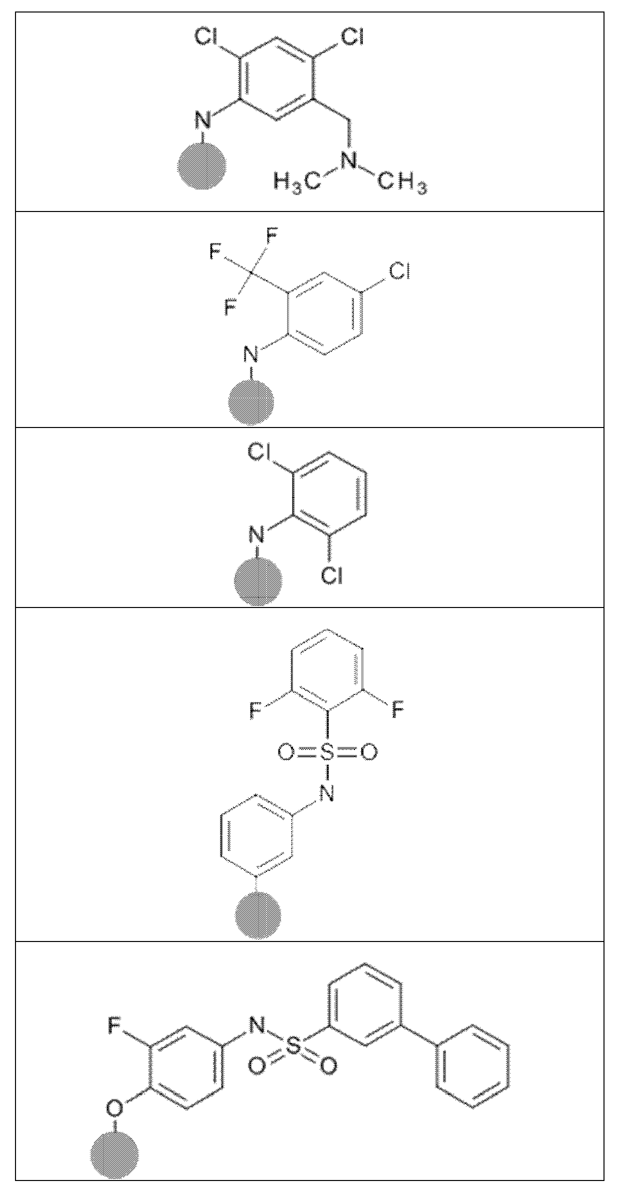

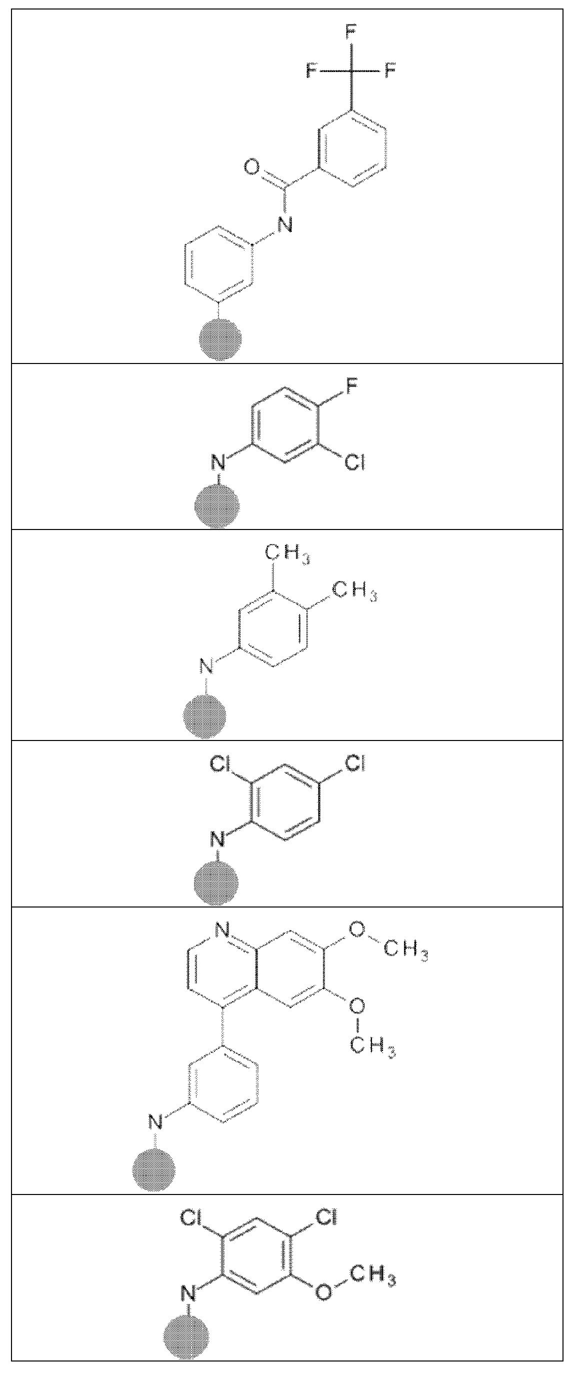

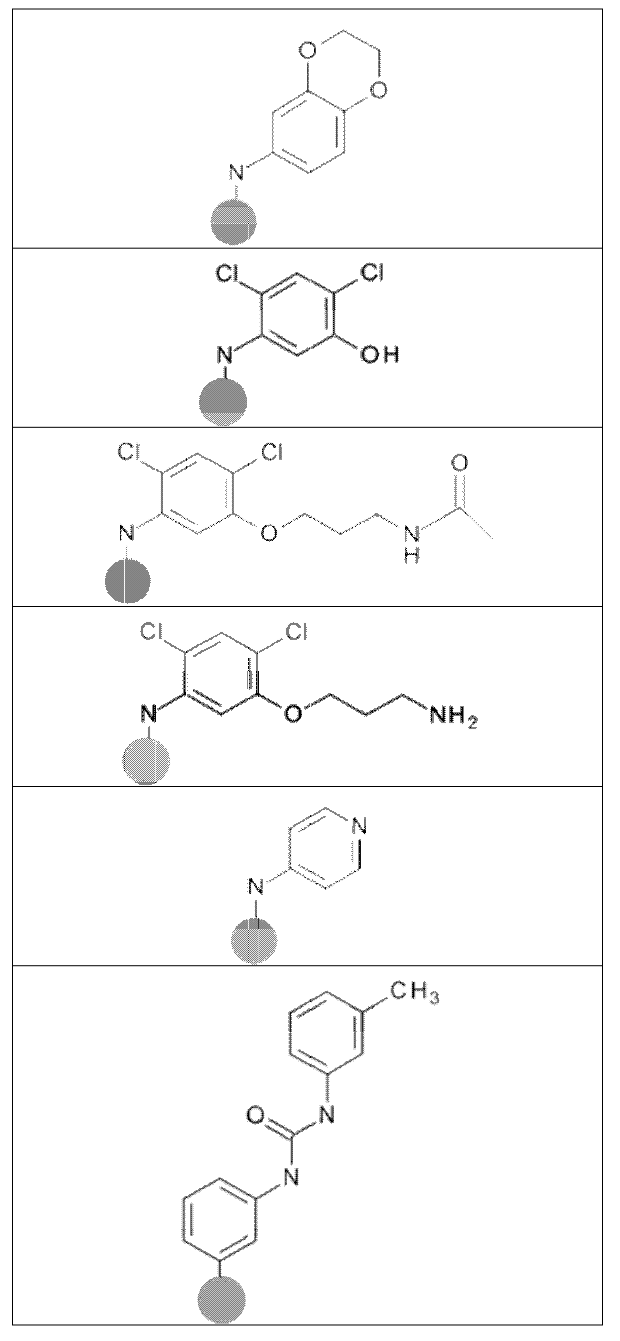

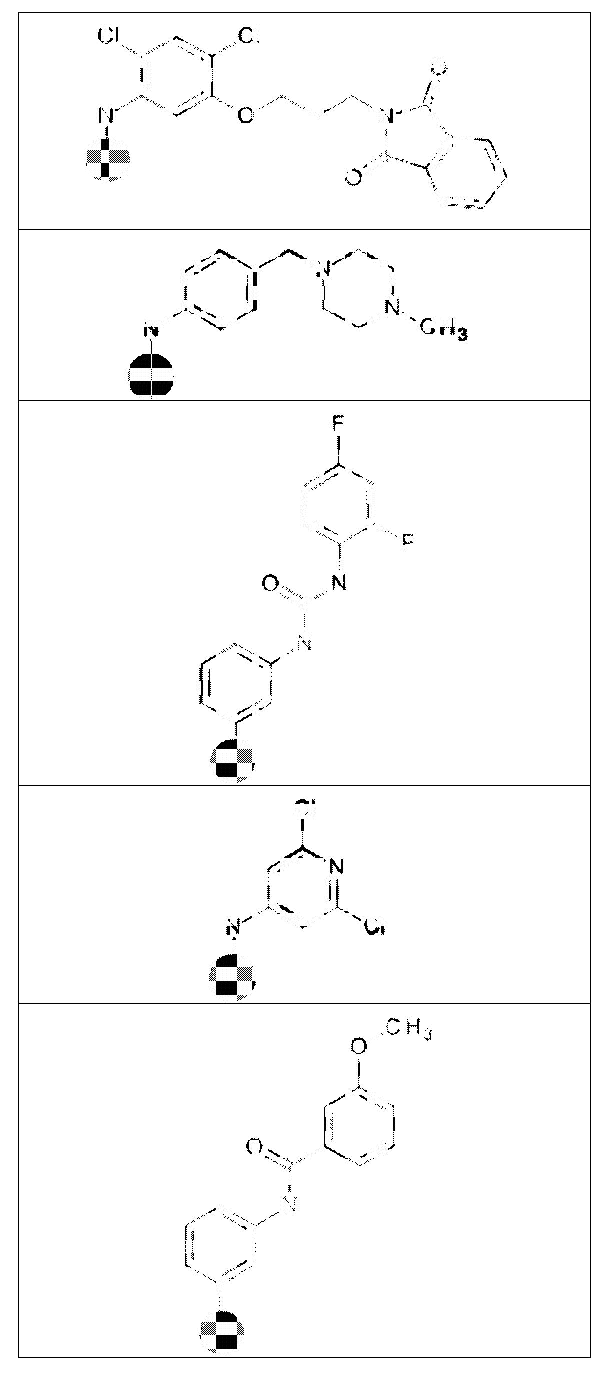

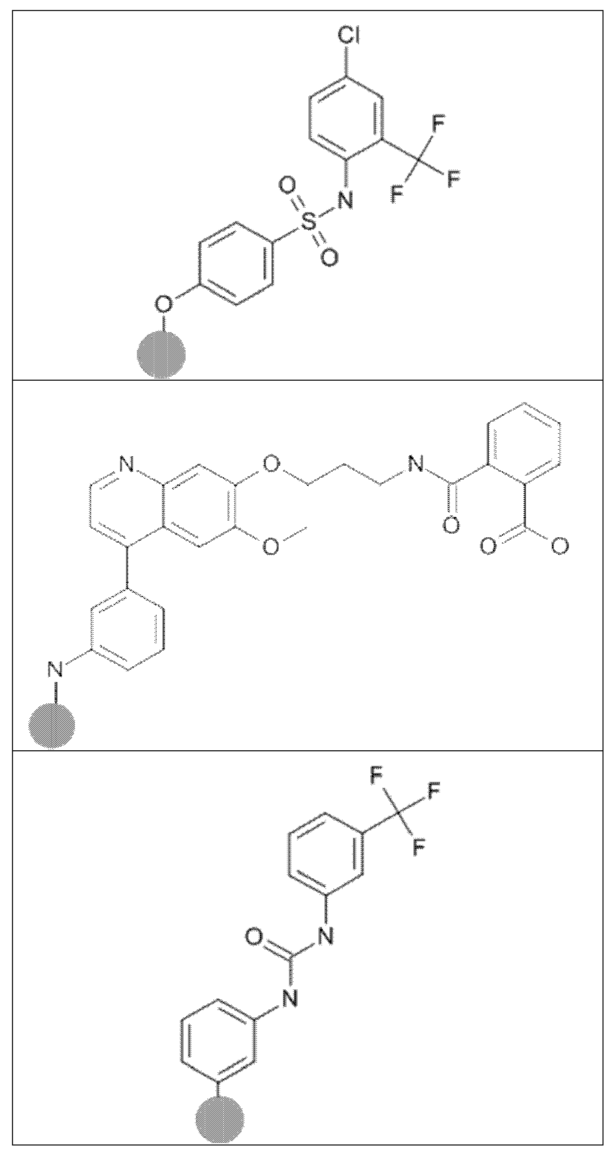

8. The insulin secretagogue according to any one of claims 1 to 7, wherein the insulin secretagogue is able to induce divalent calcium cation influx in pancreatic beta-cells.

9. The insulin secretagogue according to any one of claims 1 to 8, wherein the insulin secretagogue is able to activate voltage-dependent calcium channels in pancreatic beta-cells.

10. The insulin secretagogue according to any one of claims 1 to 9, wherein the insulin secretagogue is selected from the group consisting of:
| |
|---|
| 4-(3-aminophenyl)-7-fluoro-quinoline-2-carbonitrile |
| 5-[(8-bromo-2-cyano-4-quinolyl)amino]-2,4-dichloro-N,N-dimethyl-benzamide |
| 8-bromo-4-(2,4-dichloro-5-methoxy-anilino)quinoline-2-carbonitrile |
| 4-[4-chloro-2-(trifluoromethyl)anilino]-7-fluoro-8-methoxy-quinoline-2-carbonitrile |
| 4-[4-chloro-2-(trifluoromethyl)anilino]-6-fluoro-quinoline-2-carbonitrile |
| N-[3-(2-cyano-7-fluoro-4-quinolyl)phenyl]-2,6-difluoro-benzenesulfonamide |
| 4-[4-chloro-2-(trifluoromethyl)anilino]-8-fluoro-quinoline-2-carbonitrile |
| 4-(2,6-dichloroanilino)-7-fluoro-8-methoxy-quinoline-2-carbonitrile |
| N-[4-[[7-(3-aminopropoxy)-6-methoxy-4-quinolyl]oxy]-3-fluoro-phenyl]-3-phenyl-benzenesulfonamide |
| 4-(2,4-dichloro-5-methoxy-anilino)-6-fluoro-quinoline-2-carbonitrile |
| N-[3-(2-cyano-7-fluoro-4-quinolyl)phenyl]-3-(trifluoromethyl)benzamide |
| 4-(2,4-dichloro-5-methoxy-anilino)-7-fluoro-quinoline-2-carbonitrile |
| 4-[4-chloro-2-(trifluoromethyl)anilino]-7-fluoro-6-methoxy-quinoline-2-carbonitrile |
| 6,8-dichloro-4-(3-chloro-4-fluoro-anilino)quinoline-2-carbonitrile |
| 4-(2,4-dichloro-5-methoxy-anilino)-6-methoxy-quinoline-2-carbonitrile |
| 4-(3,4-dimethylanilino)-7-fluoro-8-methoxy-quinoline-2-carbonitrile |
| 7-(3-aminopropoxy)-4-(2,4-dichloro-5-methoxy-anilino)-6-methoxy-quinoline-3-carbonitrile |
| 4-(2,6-dichloroanilino)-8-fluoro-quinoline-2-carbonitrile |
| 4-(2,4-dichloro-5-methoxy-anilino)-8-fluoro-quinoline-2-carbonitrile |
| 7-(3-aminopropoxy)-4-(2,4-dichloroanilino)-6-methoxy-quinoline-3-carbonitrile |
| 4-(2,6-dichloroanilino)-7-fluoro-6-methoxy-quinoline-2-carbonitrile |
| 4-(2,4-dichloro-5-methoxy-anilino)-8-(trifluoromethyl)quinoline-2-carbonitrile |
| 4-[2,4-dichloro-5-(dimethylaminomethyl)anilino]-7-fluoro-quinoline-2-carbonitrile |
| 4-[3-(6,7-dimethoxy-4-quinolyl)anilino]-8-fluoro-quinoline-2-carbonitrile |
| 2,4-dichloro-5-[(2-cyano-8-fluoro-4-quinolyl)amino]-N,N-dimethyl-benzamide |
| N-[3-fluoro-4-[[6-methoxy-7-(3-morpholinopropoxy)-4-quinolyl]oxy]phenyl]-3-phenyl-benzenesulfonamide |
| 4-[3-(6,7-dimethoxy-4-quinolyl)anilino]-8-fluoro-quinoline-2-carboxamide |
| 2,4-dichloro-5-[(2-cyano-7-fluoro-4-quinolyl)amino]-N,N-dimethyl-benzamide |
| 4-(2,6-dichloroanilino)-6-fluoro-quinoline-2-carbonitrile |
| 4-(3,4-dimethylanilino)-8-fluoro-quinoline-2-carbonitrile |
| 4-(2,4-dichloroanilino)-6,7-dimethoxy-quinoline-3-carbonitrile |
| 4-(2,4-dichloroanilino)-7-[3-(dimethylamino)propoxy]-6-methoxy-quinoline-3-carbonitrile |
| 4-(2,4-dichloro-5-methoxy-anilino)-7-fluoro-8-methoxy-quinoline-2-carbonitrile |
| 4-[2,4-dichloro-5-(dimethylaminomethyl)anilino]-7-fluoro-quinoline-2-carboxamide |
| 4-(2,4-dichloro-5-methoxy-anilino)-6-methoxy-quinoline-2-carboxamide |
| N-[4-[[7-[3-(dimethylamino)propoxy]-6-methoxy-4-quinolyl]oxy]-3-fluoro-phenyl]-3-phenyl-benzenesulfonamide |
| 4-(2,4-dichloro-5-methoxy-anilino)-6-(3-morpholinopropoxy)quinoline-2-carboxamide |
| 6-(3-aminopropoxy)-4-(2,4-dichloro-5-methoxy-anilino)-7-fluoro-quinoline-2-carbonitrile |
| 6,8-dichloro-4-[2,4-dichloro-5-(dimethylcarbamoyl)anilino]quinoline-2-carboxamide |
| 4-(2,3-dihydro-1,4-benzodioxin-6-ylamino)-8-fluoro-quinoline-2-carbonitrile |
| N-[[4-(2,4-dichloro-5-methoxy-anilino)-6-fluoro-2-quinolyl]methyl]methanesulfonamide |
| 4-(2,4-dichloro-5-methoxy-anilino)-6-methoxy-quinoline-2-carboxylic acid |
| 4-[3-(6,7-dimethoxy-4-quinolyl)anilino]-8-[3-(1,3-dioxoisoindolin-2-yl)propoxy]-7-fluoro-quinoline-2-carbonitrile |
| 4-(2,4-dichloro-5-methoxy-anilino)-6-fluoro-quinoline-2-carboxamide |
| 4-(2,3-dihydro-1,4-benzodioxin-6-ylamino)-7-fluoro-6-methoxy-quinoline-2-carbonitrile |
| N-[3-[[3-cyano-4-(2,4-dichloroanilino)-6-methoxy-7-quinolyl]oxy]propyl]formamide |
| 8-bromo-4-(2,4-dichloro-5-methoxy-anilino)quinoline-2-carboxamide |
| 4-(3-chloro-4-fluoro-anilino)-8-fluoro-quinoline-2-carbonitrile |
| 8-bromo-4-[2,4-dichloro-5-(dimethylcarbamoyl)anilino]quinoline-2-carboxamide |
| N-[3-[2,4-dichloro-5-[(2-cyano-7-fluoro-4-quinolyl)amino]phenoxy]propyl]acetamide |
| 4-[5-(3-aminopropoxy)-2,4-dichloro-anilino]-7-fluoro-quinoline-2-carbonitrile |
| N-[3-[[2-cyano-4-(2,4-dichloro-5-methoxy-anilino)-7-fluoro-6-quinolyl]oxy]propyl]acetamide |
| 6-fluoro-4-(4-pyridylamino)quinoline-2-carbonitrile |
| 4-(3,4-dimethylanilino)-7-fluoro-6-methoxy-quinoline-2-carbonitrile |
| 1-[3-(2-cyano-7-fluoro-4-quinolyl)phenyl]-3-(m-tolyl)urea |
| 4-(2,4-dichloro-5-hydroxy-anilino)-6-fluoro-quinoline-2-carboxamide |
| N-(2,4-dichloro-5-methoxy-phenyl)-6-methoxy-7-(3-pyrrolidin-1-ylpropoxy)quinolin-4-amine |
| 8-fluoro-4-[4-[(4-methylpiperazin-1-yl)methyl]anilino]quinoline-2-carbonitrile |
| 4-(2,4-dichloro-5-hydroxy-anilino)-6-fluoro-quinoline-2-carboxamide |
| 7-(4-aminobutoxy)-4-(2,4-dichloro-5-methoxy-anilino)quinoline-2-carbonitrile |
| 4-[2,4-dichloro-5-[3-(1,3-dioxoisoindolin-2-yl)propoxy]anilino]-7-fluoro-6-methoxy-quinoline-2-carbonitrile |
| 4-(2,4-dichloro-5-methoxy-anilino)-7-(3-hydroxypropoxy)quinoline-2-carboxamide |
| 4-(2,4-dichloro-5-methoxy-anilino)-6-[3-(1,3-dioxoisoindolin-2-yl)propoxy]-7-fluoro-quinoline-2-carbonitrile |
| 2-(aminomethyl)-N-(2,4-dichloro-5-methoxy-phenyl)-7-fluoro-quinolin-4-amine |
| 8-bromo-4-(2,4-dichloro-5-methoxy-anilino)-6-fluoro-quinoline-2-carbonitrile |
| 4-[(2,6-dichloro-4-pyridyl)amino]-7-fluoro-8-methoxy-quinoline-2-carbonitrile |
| 1-[3-(2-cyano-7-fluoro-4-quinolyl)phenyl]-3-(2,4-difluorophenyl)urea |
| tert-butyl N-[3-[[4-[2-fluoro-4-[(3-phenylphenyl)sulfonylamino]phenoxy]-6-methoxy-7-quinolyl]oxy]propyl]carbamate |
| 2-[3-[[4-[3-[(2-cyano-8-fluoro-4-quinolyl)amino]phenyl]-6-methoxy-7-quinolyl]oxy]propylcarbamoyl]benzoic acid |
| N-[3-(2-cyano-7-fluoro-4-quinolyl)phenyl]-3-methoxy-benzamide |
| N-[4-chloro-2-(trifluoromethyl)phenyl]-4-[(2-cyano-7-fluoro-6-methoxy-4-quinolyl)oxy]benzenesulfonamide |
| 1-[3-(2-cyano-7-fluoro-4-quinolyl)phenyl]-3-[3-(trifluoromethyl)phenyl]urea |
| 7-benzyloxy-N-(2,4-dichloro-5-methoxy-phenyl)-6-methoxy-quinolin-4-amine |
| 4-(2,4-dichloro-5-methoxy-anilino)-7-fluoro-6-methoxy-quinoline-3-carbonitrile |
| 4-(2,4-dichloro-5-methoxy-anilino)-7-fluoro-6-methoxy-quinoline-2-carbonitrile |
| N-(2,4-dichloro-5-methoxy-phenyl)-2-methyl-6-(3-morpholinopropoxy)quinolin-4-amine |
| 2-(aminomethyl)-N-(2,4-dichloro-5-methoxy-phenyl)-6-fluoro-quinolin-4-amine |
| N-(2,4-dichloro-5-methoxy-phenyl)-6-methoxy-2-methyl-quinolin-4-amine |
| N-[[4-(2,4-dichloro-5-methoxy-anilino)-6-fluoro-2-quinolyl]methyl]acetamide |
| N-(2,4-dichloro-5-methoxy-phenyl)-6-methoxy-7-[3-(4-methylpiperazin-1-yl)propoxy]quinolin-4-amine |

11. The insulin secretagogue according to any one of claims 1 to 10 for use in a therapeutic method.

12. The insulin secretagogue according to any one of claims 1 to 10 for use in the treatment and prevention of diabetes.

13. The insulin secretagogue for use according to claim 12, wherein the diabetes is any form of diabetes except type I diabetes.

14. The insulin secretagogue for use according to any one of claims 12 or 13, wherein the use of sulfonylureas, meglitinides, or biguanides is contraindicated.

15. Use of the insulin secretagogue according to any one of claims 1 to 10 for (a) inducing insulin secretion in pancreatic beta-cells in vitro, (b) induce divalent calcium cation influx in pancreatic beta-cells in vitro, or (c) activating voltage-dependent calcium channels in pancreatic beta-cells in vitro.
